# EUROPEAN PATENT SPECIFICATION

(11) **EP 3 303 371 B1**
(45) Date of publication and mention of the grant of the patent: **03.05.2023**
(21) Application number: 16732949.9
(22) Date of filing: 25.05.2016
(51) Int. Cl.: C07K 14/195, C12N 9/50, C12R 1/01, C12N 1/36

(54) **ATTENUATED PISCIRICKETTSIA SALMONIS BACTERIUM**
ATTENUIERTES PISCIRICKETTSIA SALMONIS BAKTERIUM
BACTÉRIE PISCIRICKETTSIA SALMONIS ATTÉNUÉE

(30) Priority: 26.05.2015 GB 201509004
(43) Date of publication of application: 11.04.2018
(73) Proprietor: Pharmaq AS, 7863 Overhalla (NO)
(72) Inventor: BORDEVIK, Marianne, 0213 Oslo (NO); NYGAARD, Anja, 0213 Oslo (NO); TUNHEIM, Siv Haugen, 0213 Oslo (NO); FRØYSTAD-SAUGEN, Marianne, 0213 Oslo (NO); KLEVAN, Are, 0213 Oslo (NO); MAIRA, Claudia Vargas, 0213 Oslo (NO)
(74) Representative: Mannion, Sally Kim
(86) International application number: PCT/EP2016/061862
(87) International publication number: WO 2016/189067

(56) References cited:
- WO-A2-2014/198913
- DATABASE UniProt [Online] 26 November 2014 (2014-11-26), "SubName: Full=ATP-grasp domain protein {ECO:0000313|EMBL:ALB21748.1};", XP002761895, retrieved from EBI accession no. UNIPROT:A0A095BFK5 Database accession no. A0A095BFK5
- DATABASE GENBANK [Online] 26 November 2014 (2014-11-26), Millar,A.D., Gomez,F.F., Tapia,P.V., Guzman,D.A., Henriquez,V.,Sobral,B.W., Marchal,S.H. and Valdes,J.H.: "Piscirickettsia salmonis LF-89 = ATCC VR-1361 strain LF-89, whole genome shotgun sequencing project. Draft genome sequence and comparative analysis of Piscirickettsi", XP002761882, Database accession no. AMFF00000000.2 cited in the application
- M ROZAS ET AL: "Piscirickettsiosis and Piscirickettsia salmonis in fish: a review", JOURNAL OF FISH DISEASES, vol. 37, no. 3, 26 March 2014 (2014-03-26) , pages 163-188, XP055144904, ISSN: 0140-7775, DOI: 10.1111/jfd.12211

## Description

### Field of the Invention

In the broadest aspect, the present invention relates to an attenuated bacterium, its preparation, and its use in a live attenuated vaccine.

### Background

Salmon Rickettsial Syndrome, "SRS", (also known as, Piscirickettsiosis, Coho salmon septicaemia, or Huito disease) is considered to be one of the most important disease problems facing the salmon farming industry. The bacterium *Piscirickettsia salmonis* is the causative agent of SRS.

SRS continues to evolve and new outbreaks continually occur which are increasingly insidious and refractory to treatments. New outbreaks frequently show increased bacterial virulence, clinical and pathological severity and variable presentation under similar conditions of species, age and management measures.

SRS has proven very difficult to control. The use of antibiotics, both prophylactically and during early infection, may inhibit the growth of the pathogen, but failure of antibiotic treatment is common, and antibiotic treatments have been largely unsuccessful in stopping disease outbreaks.

Thus, there is a need for improved methods of controlling *P. salmonis.*

Vaccines based on live but attenuated micro-organisms (live attenuated vaccines) induce a highly effective type of immune response. Generally, such vaccines induce stronger and more durable immunity than vaccines based on an inactivated pathogen as they activate all phases of the immune system. Specifically, once an animal host has been vaccinated with a live attenuated vaccine, entry of the microbial pathogen into the host induces an accelerated recall of earlier, cell-mediated and/or humoral immunity which is able to control the further growth of the organism before the infection can assume clinically significant proportions. Inactivated vaccines (based on killed micro-organisms or fragments of micro-organisms) are less likely to be able to achieve the same magnitude and rapidity of response.

There is thus a need for an attenuated strain of *P. salmonis,* suitable for use in a vaccine. The attenuated vaccine should substantially retain the antigenic capacity of the wild-type strain in order to cause a robust immune response in the host, and thereby provide strong immunity. The vaccine should also be sufficiently avirulent to minimise undesirable pathological effects. In addition, the live attenuated vaccine strain should have substantially no likelihood of reversion to a virulent form.

The general approach for attenuating bacteria is the removal of one or more virulence factors. In most cases however, virulence factors are required in order to induce immunity, and deletion of virulence factors unavoidably impairs the immunogenic capacities of the bacterium.

It has now surprisingly been found that by mutating the *rpoD, FecR, ATP-grasp domain protein,* and *FtsH* gene products, an attenuated *P. salmonis* bacterium can be produced, without impairing the viability or immunogenicity of such bacteria in vivo. By mutating a number of genes in parallel, the likelihood of reversion to a virulent form is minimised. Moreover, these genes were not previously known to relate to virulence factors, and it is therefore surprising that they have now been found to affect bacterial pathogenicity. This also offers the further advantage that the attenuated bacterium demonstrates substantially the same level of immunogenicity as wild-type strains. The disclosed bacterium has therefore surprisingly been found to be extremely suitable for use in the preparation of live attenuated vaccines.

### Summary

According to a first aspect there is provided, an attenuated *Piscirickettsia salmonis* bacterium comprising a mutation in the amino acid sequence of each of the *rpoD, FecR, ATP-grasp domain protein,* and *FtsH* gene products, the mutations comprising:
a) an arginine to cysteine mutation at position 473 of the *rpoD* gene product provided as Seq. ID No. 17;
b) a premature stop codon at the position corresponding to residue 83 of the *FecR* gene product provided as Seq. ID No. 26;
c) a serine to proline mutation at position 184 of *the ATP-grasp domain protein* gene product provided as Seq. ID No. 40; and,
d) a methionine to isoleucine mutation at position 191 of the *FtsH* gene product provided as Seq. ID No. 54.

Thus, the attenuated bacterium of the present invention comprises a mutation in the amino acid sequence of each of the *rpoD, FecR, ATP-grasp domain protein,* and *FtsH* gene products.

The attenuated bacterium has a reduced virulence relative to wild-type *P. salmonis.* The attenuated bacterium is preferably avirulent and does not induce any symptoms of Salmon Rickettsial Syndrome when administered to fish.

The attenuated bacterium preferably does not revert to a virulent strain after serial passage in fish. For example, the attenuated bacterium preferably does not revert to a virulent strain after 2, 3, 4, 5, or 6 passages in fish.

The attenuated bacterium is preferably capable of inducing immunological protection against Salmon Rickettsial Syndrome when administered to fish. Indeed, the attenuated bacterium preferably provides protection to fish against SRS following subsequent challenge with a virulent strain of *P. salmonis.* Preferably, when measured in terms of accumulated mortality, the attenuated bacterium provides more than 40%, more than 50%, more than 60%, or more than 80% protection against SRS. Preferably, when measured in terms of accumulated mortality, the attenuated bacterium provides 100% protection against SRS.

The mutations in each of the *rpoD, FecR, ATP-grasp domain protein,* and *FtsH* genes of the attenuated bacterium, which underlie the mutations in the corresponding gene products, may be non-reverting mutations.

The mutations in the amino acid sequence of the *rpoD, FecR, ATP-grasp domain protein,* and *FtsH* gene products are mutations relative to the sequence of the corresponding LF-89 wild-type protein, as derived from the LF-89 genomic sequence that is available under the GenBank accession no. AMFF00000000.2, and as provided as Seq. ID No.s 17, 26, 40, and 54, respectively.

The amino acid residue numbers given throughout are defined on the basis of the sequences of the corresponding LF-89 wild-type proteins, as shown in Example 2 (and given as Seq. ID No.s 17, 26, 40, 54), and derived from the LF-89 genomic sequence that is available under the GenBank accession no. AMFF00000000.2.

The attenuated bacterium may be the strain PHARMAQ 001 deposited with the European Collection of Cell Cultures, Public health England, Culture Collections, Porton Down, Salisbury SP4 OJG, United Kingdom, on 09th October 2014 with accession number 14100901.

According to a second aspect, the invention provides a live, attenuated vaccine composition comprising:
(a) an attenuated *Piscirickettsia salmonis* bacterium of the first aspect; and
(b) a pharmaceutically acceptable carrier or diluent.

The live, attenuated vaccine composition may be in freeze-dried form.

According to a third aspect, the invention provides a method of producing an attenuated bacterium in accordance with the first aspect. The method comprises:
1) subjecting an initial population of *P. salmonis* bacteria to attenuating conditions to produce a putatively attenuated bacterial population;
2) identifying clones of the putatively attenuated bacterial population that have mutations in the amino acid sequences of all of the *rpoD, FecR, ATP-grasp domain protein,* and *FtsH* gene products; and then,
3) identifying and selecting clones that have mutations in the amino acid sequence of all of the *rpoD, FecR, ATP-grasp domain protein,* and *FtsH* gene products and that also exhibit reduced virulence relative to wild-type bacteria of the genus Piscirickettsia.

According to a fourth aspect, the invention provides a vaccine composition according to the second aspect for use in raising an immune response in a fish. The use comprises administering to the fish a vaccine composition according to the second aspect comprising an attenuated *Piscirickettsia salmonis* bacterium of the first aspect.

According to a fifth aspect, the invention provides a vaccine composition according to the second aspect for use in prevention or treatment against Salmon Rickettsial Syndrome. The use comprises administering to a fish an immunologically-effective amount of said vaccine composition comprising an attenuated *Piscirickettsia salmonis* bacterium of the first aspect.

According to a sixth aspect, the invention provides an attenuated *Piscirickettsia salmonis* bacterium of the first aspect, for use in a method of vaccinating a fish.

According to a seventh aspect, the invention provides an attenuated *Piscirickettsia salmonis* bacterium of the first aspect, for use in a method of vaccinating a fish against Salmon Rickettsial Syndrome.

The PHARMAQ 001 strain of *Piscirickettsia salmonis* can be distinguished from other strains such as wild-type strains by distinguishing between wild-type and mutant alleles of a *Piscirickettsia salmonis* single nucleotide polymorphism (SNP) located at the position corresponding to:
- residue number 1417 of Seq. ID No. 1 (in the *rpoD* gene);
- residue number 247 of Seq. ID No. 4 (in the *FecR gene*);
- residue number 550 of Seq. ID No. 7 (in *the ATP-grasp domain protein* gene); or,
- residue number 573 of Seq. ID No. 10 (in the *FtsH* gene).

The method of distinguishing comprises:
i) amplifying by PCR the region of the nucleotide sequence containing the SNP;
ii) including in the PCR reaction mix a nucleic acid probe having a sequence complementary to one allele of the SNP, the probe comprising a detectable marker; and
iii) analysing the PCR product for the presence of the marker, wherein the presence of the marker is indicative of the presence of the allele.

The method of distinguishing may further comprise including in the PCR reaction mix a first nucleic acid probe having a sequence complementary to the wild-type allele of the SNP, and a second nucleic acid probe having a sequence complementary to the mutant allele of the SNP, the first and second probes comprising different detectable markers.

The probe or, when two or more probes are used, one or more of the probes, may comprise a 10-40 nucleotide subsequence of:
- Seq. ID No. 1, the subsequence including residue number 1417;
- Seq. ID No. 4, the subsequence including residue number 247;
- Seq. ID No. 7, the subsequence including residue number 550; or,
- Seq. ID No. 10, the subsequence including residue number 573.

Specifically, the probe or probes may consist of or comprise Seq. ID No. 68, 69, 72, 73, 76, or 77.

A PCR primer pair may be used in the method to amplify a region of at least 50 nucleotides in length of the subsequence of:
- Seq. ID No. 1, the subsequence including residue number 1417;
- Seq. ID No. 4, the subsequence including residue number 247;
- Seq. ID No. 7, the subsequence including residue number 550; or,
- Seq. ID No. 10, the subsequence including residue number 573.

Specifically, the PCR primer pair may be or comprise Seq. ID No. 67 and 70, 71 and 74, or 75 and 78.

### Detailed Description

### P. salmonis

SRS is caused by the gram-negative bacterium, *Piscirickettsia salmonis.* This was the first "rickettsia-like" bacterium to be recognized as a pathogen of fish.

*P. salmonis* is generally non-motile, obligate intracellular bacterium, pleomorphic but predominately coccoid, and 0.5-1.5 µm in diameter. It is currently placed in the class Gammaproteobacteria; order Thiotrichales; and family Piscirickettsiacaea, and has a closer relationship to, e.g., Legionella and Coxiella, than to members of the genera Rickettsia. The bacterium replicates by binary fission within membrane bound cytoplasmic vacuoles in fish cell lines and in the cells of tissues throughout infected fish. The bacteria occur either singularly or in groups, giving the vacuole the appearance of a morula. When *P. salmonis* is examined by electron microscopy, the bacterium displays the typical protoplasmic structure of a prokaryote and the cell wall of a gram-negative bacterium.

The genome of *P. salmonis* strain LF-89 has been sequenced and published on at least three separate occasions (see, for example, Eppinger et al. Genome Announc. November/December 2013 vol. 1 no. 6), and is available via DDBJ/EMBL/GenBank under the accession no.s AMGC00000000.1, AMFF00000000.2, and ASSK00000000.2. Unless otherwise indicated, the LF-89 sequence used in the present application is the sequence available under the accession no. AMFF00000000.2. Specifically, unless otherwise indicated, for the purposes of genetic and protein sequence comparison in particular, this LF-89 sequence that is available under the GenBank accession no. AMFF00000000.2 is considered to represent the sequence of wild-type *P. salmonis,* and references to the wild-type in this context should be interpreted accordingly. The genomes of *P. salmonis* strains EM-90 (NCBI Reference Sequence: NZ_JRHP00000000.1), A1-15972 (NCBI Reference Sequence: NZ_JRAV00000000.2), and B1-32597 (NCBI Reference Sequence: NZ JRAD00000000.2) have also been published.

### Attenuated Bacterium

The attenuated *P. salmonis* bacterium of the invention is attenuated by means of a mutation in each of the *rpoD, FecR, ATP-grasp domain protein,* and *FtsH* genes. Specifically, the attenuated bacterium comprises genetic mutations which result in mutations in the amino acid sequence of each of the *rpoD, FecR, ATP-grasp domain protein,* and *FtsH* gene products, relative to the sequence of the wild-type proteins.

For the purposes of the invention, the term "gene product" is specifically considered to refer to the protein resulting from the expression of a gene.

For the purposes of the invention, a "mutation" is considered to be any alteration in the gene or protein sequence relative to the wild-type sequence. Genetic mutations that are of interest are those that result in a mutation (i.e. alteration) in the resulting amino acid sequence of the gene product relative to the wild-type amino acid sequence. Each of the mutations in the *rpoD, FecR, ATP-grasp domain protein,* and *FtsH* genes can be any type of mutation, including an insertion, a deletion, a substitution, or any combination of these, provided that the mutation leads to a change in the amino acid sequence of each of the *rpoD, FecR, ATP-grasp domain protein,* and *FtsH* gene products, relative to the wild-type protein sequence.

A functional gene product is a protein having the functional characteristics of the wild-type protein. A *rpoD, FecR, ATP-grasp domain protein,* or *FtsH* gene product that is at least partially defective in at least one of its functions is considered to be an attenuated gene product. Any mutation resulting in an attenuated gene product is considered to be an attenuating mutation. The mutations in the amino acid sequence of each of the *rpoD, FecR, ATP-grasp domain protein,* and *FtsH* gene products, relative to the wild-type proteins, are preferably attenuating mutations.

Attenuating mutations in the *rpoD, FecR, ATP-grasp domain protein,* and *FtsH* gene products may knock-out the function of the gene product partially or completely. The partial or total functional knock-out may be achieved, for example, by making a mutation that results in the synthesis of non-functional or partially functional polypeptide. For example, the mutation in the amino acid sequence may comprise the insertion of a stop codon, or may result in the incorporation of an amino acid that is physically or chemically dissimilar to the wild-type residue. Such mutations may result in the production of a truncated protein, a misfolded protein, or a chemically inactive protein, for example.

The mutations in each of the *rpoD, FecR, ATP-grasp domain protein,* and *FtsH* genes, which result in mutated gene products, are preferably non-reverting mutations. These are mutations that show essentially no reversion back to the wild-type when the bacterium is used as a vaccine.

The possibility of reversion of the bacterium to full virulence is also eliminated by the fact the bacterium contains attenuating mutations in four independent genes.

### Attenuated Genes

The gene *rpoD* encodes RNA polymerase sigma factor, which is an initiation factor involved in promoting the attachment of RNA polymerase to specific transcription initiation sites. The *rpoD* gene product is believed to be involved in in the regulation of essential housekeeping genes. For the avoidance of doubt, the wild-type *P. salmonis rpoD* gene sequence that is mutated in the present invention is given as Seq. ID No. 1, and the gene can be identified using the PCR primers of Seq. ID No.s 2 and 3. The amino acid sequence of the full-length wild-type protein is given in Seq. ID No.s 14-17. The attenuated bacterium of the invention comprises a mutation in the amino acid sequence of the *rpoD* gene product, relative to the sequence of the wild-type protein, which is exemplified by the LF-89 sequence, given as Seq. ID No. 17.

The gene *FecR* encodes an iron dicitrate transport regulator. The *FecR* gene product is believed to be involved in regulating a number of genes involved in the uptake of iron and citrate. For the avoidance of doubt, the wild-type *P. salmonis FecR* gene sequence that is mutated in the present invention is given as Seq. ID No. 4, and the gene can be identified using the PCR primers of Seq. ID No.s 5 and 6. The amino acid sequence of the full-length wild-type protein is given in Seq. ID No.s 26-29. The attenuated bacterium of the invention comprises a mutation in the amino acid sequence of the *FecR* gene product, relative to the sequence of the wild-type protein, which is exemplified by the LF-89 sequence, given as Seq. ID No. 26.

The *ATP-grasp domain protein* gene encodes a protein with similarity to an alpha-L-glutamate ligase-related protein found in Pseudomonas (GenBank accession No. AP 014655.1). For the avoidance of doubt, the wild-type *P. salmonis ATP-grasp domain protein* gene sequence that is mutated in the present invention is given as Seq. ID No. 7, and the gene can be identified using the PCR primers of Seq. ID No.s 8 and 9. The amino acid sequence of the full-length wild-type protein is given in Seq. ID No.s 40-43. The attenuated bacterium of the invention comprises a mutation in the amino acid sequence of the *ATP-grasp domain protein* gene product, relative to the sequence of the wild-type protein, which is exemplified by the LF-89 sequence, given as Seq. ID No. 40.

The gene *FtsH* encodes an ATP-dependent zinc metalloprotease, which acts as a processive, ATP-dependent zinc metallopeptidase for both cytoplasmic and membrane proteins. The *FtsH* gene product is also believed to play a role in the quality control of integral membrane proteins. For the avoidance of doubt, the wild-type *P. salmonis FtsH* gene sequence that is mutated in the present invention is given as Seq. ID No. 10, and the gene can be identified using the PCR primers of Seq. ID No.s 11 and 12. The amino acid sequence of the full-length wild-type protein is given in Seq. ID No.s 54-57. The attenuated bacterium of the invention comprises a mutation in the amino acid sequence of the *FtsH gene* product, relative to the sequence of the wild-type protein, which is exemplified by the LF-89 sequence, given as Seq. ID No. 54.

The genes may be expressed at wild-type levels, but mutated so that the gene products have a different amino acid sequence to that found in wild-type strains. The genetic mutation may result in a deletion, an insertion, and/or a substitution of one or more amino acids in the gene product. The genetic mutation may result in full-length or substantially full-length gene products, or truncated gene products. The mutation may be a point mutation, affecting just one amino acid, or may affect more than one amino acid residue, such as, for example, affecting 2-20 residues, 3-15 residues, 4-12 residues, or 5-10 residues.

In the attenuated *Piscirickettsia salmonis* bacterium of the first aspect, the *rpoD* gene is mutated resulting in the replacement of arginine with cysteine at position 473 in the amino acid sequence of the gene product. As a result of this mutation, the protein encoded by the mutated *rpoD* gene has different functional properties to those of the wild-type protein.

Also in the attenuated *Piscirickettsia salmonis* bacterium of the first aspect the *FecR* gene is mutated resulting in the insertion of a premature stop codon, for example, in the position of residue 83, and therefore the production of a truncated gene product, having different functional properties to those of the wild-type protein.

Also in the attenuated *Piscirickettsia salmonis* bacterium of the first aspect the *ATP-grasp domain protein* gene is mutated resulting in the replacement of serine with proline at position 184 in the amino acid sequence of the gene product. As a result, the mutated ATP-grasp domain protein has different functional properties to those of the wild-type protein.

Also in the attenuated *Piscirickettsia salmonis* bacterium of the first aspect the *FtsH* gene is mutated resulting in the replacement of methionine with isoleucine at position 191 in the amino acid sequence of the gene product. As a result, the protein encoded by the mutated *FtsH* gene has different functional properties to those of the wild-type protein.

All four, of the specific point mutations described above in the *rpoD, FecR, ATP-grasp domain protein,* and *FtsH* genes are present in combination in the attenuated *Piscirickettsia salmonis* bacterium of the first aspect.

The bacterium preferably contains only defined mutations, which are fully characterised. It is less preferred to use a bacterium which has uncharacterised mutations in its genome as a vaccine because there would be a risk that the uncharacterised mutations may confer properties on the bacterium that cause undesirable side-effects.

### Production of Attenuated P. salmonis

Methods for identifying and/or producing attenuated *P. salmonis* clones include subjecting an initial population of *P. salmonis* bacteria to attenuating conditions, thereby producing a putatively attenuated bacterial population.

The "initial population of *P. salmonis* bacteria" can be any quantity of *P. salmonis* bacteria. The bacteria are wild-type *P. salmonis* bacteria. A number of strains of *P*. *salmonis* have been isolated following outbreaks of SRS. Any of these isolated strains would potentially be suitable as a starting population for producing a putatively attenuated bacterial population, including the following strains: AL 10 016, AL 10 008, AL 20 218, AL 20 219, AL 20 223, AL 20 220, AL 20 470, AL 20 471, AL 20 455, AL 20 222, A1-15972, B1-32597, LF-89, EM-90. References to wild-type *P. salmonis* may refer to any of these strains. Preferably, however, references to wild-type *P. salmonis* refer to any of strains A1-15972, B1-32597, LF-89, or EM-90, such as in particular, strains A1-15972, LF-89, or EM-90. Unless otherwise indicated, however, for the specific purposes of genetic and protein sequence comparison, the LF-89 sequence that is available under the GenBank accession no. AMFF00000000.2 is considered to represent the sequence of wild-type *P. salmonis,* and references to the wild-type in this context should be interpreted accordingly.

The bacteria used as a starting population for producing a putatively attenuated bacterial population may alternatively contain one or more mutations relative to the wild-type, or other strain.

Preferably, the bacteria in the initial population are clonally identical or substantially clonally identical. In other words, the bacteria are preferably all derived from a single parental *P. salmonis* bacterial cell and/or have identical or substantially identical genotypic and/or phenotypic characteristics.

The term "attenuating conditions" refers to any condition or combination of conditions which has or have the potential for introducing one or more genetic changes (i.e., mutations) into the genome of a *P. salmonis* bacterium. Exemplary attenuating conditions include, for example, passaging bacteria in culture, transforming bacteria with a genome-insertable genetic element such as a transposon (e.g., a transposon that randomly inserts into the *P. salmonis* genome), exposing bacteria to one or more mutagens (e.g., chemical mutagens or ultraviolet light), and any other suitable methods.

Indeed, the attenuating mutations may be introduced by any suitable method.

A possibility to introduce a mutation at a predetermined site, deliberately rather than randomly, is offered by recombinant DNA-technology. Such a mutation may be an insertion, a deletion, a replacement of one or more nucleotides, or any combination of these, with the only proviso that the genetic mutation leads to a mutation in the amino acid sequence of the resulting gene product.

For example, one possible method includes cloning the DNA sequence of the wild-type gene into a vector, such as a plasmid, and inserting a selectable marker into the cloned DNA sequence or deleting a part of the DNA sequence, resulting in its inactivation. A deletion may be introduced by, for example, cutting the DNA sequence using restriction enzymes that cut at two points in or just outside the coding sequence and ligating together the two ends in the remaining sequence. A plasmid carrying the inactivated DNA sequence can be transformed into the bacterium by known techniques such as electroporation and conjugation. It is then possible by suitable selection to identify a mutant wherein the inactivated DNA sequence has recombined into the chromosome of the bacterium and the wild-type DNA sequence has been rendered non-functional by homologous recombination.

One or more further mutations may be introduced into the bacteria to generate strains containing mutations in genes in addition to those in the *rpoD, FecR, ATP-grasp domain protein,* and *FtsH* genes.

When bacterial cells are attenuated by passaging in vitro, the cells may be passaged any number of times, such as for example, at least 10, 20, 40, 60, 80, 100, 120, or more times in vitro.

The initial population of *P. salmonis,* after being subjected to attenuating conditions, is referred to as a putatively attenuated bacterial population. Individual clones of the putatively attenuated bacterial population can be obtained by standard microbiological techniques including, for example, serially diluting the cells and plating out individual cells on appropriate media.

Once obtained, the individual clones of the putatively attenuated bacterial population are assayed for mutations in each of the *rpoD, FecR, ATP-grasp domain protein,* and *FtsH* genes. The mutated gene sequences are then analysed to determine whether the resulting amino acid sequences have any mutations. Mutations in the amino acid sequences of the gene products are considered to be any differences in the amino acid sequences compared to the wild-type *P. salmonis* sequence.

Any suitable method may be used to determine whether a putatively attenuated *P*. *salmonis* bacterium exhibits mutations in each of the *rpoD, FecR, ATP-grasp domain protein,* and *FtsH* genes, and consequently any mutations in the amino acid sequence of any of the *rpoD, FecR, ATP-grasp domain protein,* and *FtsH* gene products.

One method by which mutations in these genes may be identified is by amplifying and sequencing portions of the genes. Any suitable PCR method may be used to amplify portions of the genes and pairs of PCR primers suitable for amplifying specific portions of the *rpoD, FecR, ATP-grasp domain protein,* and *FtsH* genes are given in Seq. ID No.s: 2 and 3; 5 and 6; 8 and 9; and 11 and 12, respectively. The amino acid sequence of the gene product can be determined from the genetic sequence using any suitable computational tool.

For the avoidance of doubt, the primers given in Seq. ID No.s: 2 and 3; 5 and 6; 8 and 9; and 11 and 12, may also be used to identify the genes referred to as *rpoD, FecR, ATP-grasp domain protein,* and *FtsH* respectively.

The portions of the genes amplified using the PCR primers of Seq. ID No.s: 2 and 3; 5 and 6; 8 and 9; and 11 and 12 are regions of the genes, which when mutated, have been found to be particularly associated with the attenuation of the bacteria. Specifically, differences between the amino acid sequences of these portions of the *rpoD, FecR, ATP-grasp domain protein,* and *FtsH* gene products, and the wild type sequences, may be indicative of attenuating mutations in the gene products. Therefore, references to mutations in the amino acid sequence of the *rpoD, FecR, ATP-grasp domain protein,* and *FtsH* gene products preferably refer to the presence of mutations in the amino acid sequence of those portions of the gene products corresponding to the regions that may be amplified by the PCR primers of Seq. ID No.s: 2 and 3; 5 and 6; 8 and 9; and 11 and 12.

Differences in the amino acid sequences of the *rpoD, FecR, ATP-grasp domain protein,* and *FtsH* gene products between the putatively attenuated and wild-type *P. salmonis* bacteria may in some cases not be accompanied by reduced function of the gene product. For the purposes of the invention, such mutations are not considered to be attenuating mutations. Non-attenuating mutations may be found in the portions of the gene products corresponding to the regions amplified using the PCR primers of Seq. ID No.s: 2 and 3; 5 and 6; 8 and 9; and 11 and 12, or elsewhere in the genes.

Some (such as 1, 2, or 3) of the *rpoD, FecR, ATP-grasp domain protein,* and *FtsH* gene products may include attenuating mutations, while the remainder of the *rpoD, FecR, ATP-grasp domain protein,* and *FtsH* gene products contain non-attenuating mutations.

The clones that have been identified as having mutations in the amino acid sequence of each of the *rpoD, FecR, ATP-grasp domain protein,* and *FtsH* gene products, relative to the wild-type sequence, are then tested for virulence.

Individual clones that are identified as having mutations in the amino acid sequence of each of the *rpoD, FecR, ATP-grasp domain protein,* and *FtsH* gene products can be tested for virulence by any suitable method. For example, the attenuated bacteria may be administered to an animal that is susceptible to infection by the wild-type version of the bacterium, and the presence and severity of disease determined.

In the present context, "an animal that is susceptible to infection by a wild-type *P*. *salmonis* bacterium" is an animal that shows at least one clinical symptom after being challenged with a wild-type *P. salmonis* bacterium. Such symptoms are known to persons of ordinary skill in the art. For example, in the case of a putatively attenuated *P. salmonis* strain that exhibits mutations in the amino acid sequence of each of the *rpoD, FecR, ATP-grasp domain protein,* and *FtsH* gene products, the strain can be administered to, for example, salmon (which are normally susceptible to infection by wild-type *P. salmonis*)*.* Clinical symptoms of SRS in salmon are known to the skilled person.

The symptoms investigated may include the accumulated mortality of a population. If the accumulated mortality is lower in animals challenged with the putatively attenuated *P. salmonis* strain, compared to fish that have been infected with a wild-type *P*. *salmonis* strain, then the putatively attenuated *P. salmonis* strain is deemed to have reduced virulence.

The symptoms investigated may include the presence or accumulation or *P. salmonis* genomes in tissue samples taken from animals challenged with the putatively attenuated *P. salmonis* strain. If the presence of *P. salmonis* DNA is reduced compared to fish that have been infected with a wild-type *P. salmonis* strain, then the putatively attenuated *P. salmonis* strain is deemed to have reduced virulence.

Thus, if the putatively attenuated *P. salmonis* strain, when administered to salmon, results in fewer and/or less severe symptoms when compared to fish that have been infected with a wild-type *P. salmonis* strain, then the putatively attenuated *P. salmonis* strain is deemed to have "reduced virulence". Any degree of reduction in any relevant symptoms will identify the putatively attenuated strain as having reduced virulence.

For the purposes of the invention, any strain that is found to have a reduced virulence is considered to be an attenuated strain. Preferably, the putatively attenuated strain is avirulent.

Clones that exhibit mutations in the amino acid sequence of the live, attenuated *P*. *salmonis* clone of the present invention in each of the *rpoD, FecR, ATP-grasp domain protein,* and *FtsH* gene products, and that also exhibit reduced virulence relative to wild-type *P. salmonis* are identified as attenuated *P. salmonis* clones of the present invention.

An exemplary, live, attenuated *P. salmonis* clone of the present invention, which exhibits non-reverting genetic mutations resulting in mutations in the amino acid sequence of each of the *rpoD, FecR, ATP-grasp domain protein,* and *FtsH* gene products is the strain designated PHARMAQ 001.

Specifically, relative to wild-type *P. salmonis,* PHARMAQ 001 has been found to have mutations located at positions corresponding to:
- residue number 1417 of Seq. ID No. 1 (in the *rpoD* gene);
- residue number 247 of Seq. ID No. 4 (in the *FecR gene*);
- residue number 550 of Seq. ID No. 7 (in the *ATP-grasp domain protein* gene); and,
- residue number 573 of Seq. ID No. 10 (in the *FtsH* gene).

For the purpose of the present disclosure, the mutations found in the *P. salmonis* strain PHARMAQ 001 are considered to represent single nucleotide polymorphisms (SNP). Various methods of distinguishing between SNP alleles are known to the skilled person, and can be used to determine whether a given strain is PHARMAQ 001. In particular, various different methods have been developed for the detection of specific alleles, or DNA sequence variants, at the same locus by polymerase chain reaction. For example, suitable methods may be based on the use of PCR primers with a 3' end specific for one of the allelic variants, or on the use of nucleic acid probes having a sequence complementary to the sequence of one particular individual allelic variant.

PHARMAQ 001 has been deposited with the European Collection of Cell Cultures, Public health England, Culture Collections, Porton Down, Salisbury SP4 OJG, United Kingdom, on 09^{th} October 2014 and was assigned accession number 14100901.

### Vaccine

A vaccine comprising the attenuated bacterium of the first aspect may be formulated using known techniques.

It has now surprisingly been found that an attenuated *P. salmonis* bacterium having a combination of mutations in the the amino acid sequences of the *rpoD, FecR, ATP-grasp domain protein,* and *FtsH* gene products gives a vaccine having superior properties for at least two reasons.

Firstly, due to the presence of multiple mutations in four independent genes there is a significantly reduced chance of reversion of attenuation of the bacterium. Therefore, the bacterium can survive in the vaccinated host for a long time and at high levels, resulting in better protection.

Secondly, the attenuated bacterium of the first aspect does not cause reduced immunogenicity compared to wild type strains because antigens important for immunogenicity are still expressed.

The vaccine composition comprises a live, attenuated *P. salmonis* bacterium of the first aspect and a pharmaceutically acceptable carrier.

Examples of pharmaceutically acceptable carriers or diluents useful in the present invention include water, a preservative, culture medium, stabilisers such as SPGA, carbohydrates (e.g. sorbitol, mannitol, starch, sucrose, glucose, dextran), proteins such as albumin or casein, protein containing agents such as bovine serum or skimmed milk, and buffers (e.g. phosphate buffer).

The vaccine may or may not comprise an adjuvant. Adjuvants are non-specific stimulators of the immune system. They enhance the immune response of the host to the vaccine. Examples of adjuvants known in the art are Freunds Complete and Incomplete adjuvant, vitamin E, non-ionic block polymers, muramyldipeptides, ISCOMs (immune stimulating complexes), Saponins, mineral oil, vegetable oil, and Carbopol.

Vaccine formulations comprising the attenuated bacterium of the first aspect can be prepared in the form of a suspension or in a lyophilized form or, alternatively, in a frozen form. If the formulation is to be frozen, glycerol or other similar agents may be included in the formulation to enhance the stability of the bacterium when frozen.

Reconstitution is advantageously effected in a buffer at a suitable pH to ensure the viability of the bacteria.

The combined administration of several vaccines is desirable, in order to save time, effort, and money. Preferably, vaccine formulations comprising the attenuated bacterium of the first aspect may be used together with other vaccines, such as, for example, an inactivated, oil adjuvanted vaccine. The vaccines may be administered together, for example, in a single composition, or separately.

### Vaccinated Species

The animal to which the vaccine comprising the attenuated bacterium of the first aspect is administered is preferably a fish. The vaccine may be administered to any species of fish that is susceptible to SRS infection.

Of particular note, the vaccine is suitable for treating fish of the order Samoniformes. For example, the claimed formulation may be used to treat salmon such as Atlantic and Pacific salmon, such as Coho salmon, and trout such as rainbow trout and brown trout.

### Method of Vaccination

The present invention includes a live attenuated vaccine composition according to the second aspect for use in methods of vaccinating fish against *P. salmonis* infection, such as SRS.

The methods according to this aspect of the invention comprise administering to a fish an immunologically-effective amount of a vaccine composition according to the second aspect comprising a live, attenuated *P. salmonis* bacterium of first aspect of the invention. The expression "immunologically-effective amount" means the amount of vaccine composition required to invoke the production of protective levels of immunity in a host upon vaccination.

The vaccine composition may be administered to the host in any manner known in the art. In particular, the vaccine formulation may be suitable for parenteral administration, such as by intraperitoneal injection.

An infection caused by a microorganism, especially a pathogen, may therefore be prevented by administering an effective dose of a vaccine prepared according to the invention.

The dosage of the vaccine employed will be dependent on various factors including the size and weight of the host, the type of vaccine formulated, and the formulation.

For example, a dosage for Atlantic salmon, Coho salmon or rainbow trout, with an average weight of 25-30 grams, may comprise the administration of from 1×10² to 1×10¹⁰, 1×10³ to 1×10⁹, or 1×10⁴ to 1×10⁸ TCID₅₀ per fish, such as from 1×10⁴ to 1×10⁷ TCID₅₀ per fish. As the skilled person will appreciate, the preferred dosage may depend on the age, weight and type of fish to be vaccinated, and the mode of administration. For example, dosages may need to be increased for larger, more robust fish, and decreased for smaller, more delicate fish.

### Examples

The invention will now be explained in further detail in the following Examples, which demonstrate the development of the claimed live, attenuated bacterium, and its use in a vaccine.

### Example 1: Isolation of the PHARMAQ 001 strain and attenuation of the strain.

The *P. salmonis* strain used as the starting bacterial population for the production of an attenuated bacterium was a strain originally isolated from an outbreak of SRS in Atlantic salmon in the X region in Chile.

The isolate was for the six first passages cultivated in the presence of eukaryotic cells.

For the next passages until passage 104, the *P. salmonis* isolate was cultivated in cell free insect cell medium at 20°C. To secure a homogenous culture, the passage 104 culture was serially diluted in insect cell medium and seeded into 96 well cell culture plates. Bacteria grown in chosen wells were further passed into new wells at an early stage when it was most likely that the growth originated from a single bacterium.

After a total of 111 passages, one clone from the wells was inoculated into a spinner flask and cultivated in insect cell medium. This passage was used as the origin of the putatively attenuated bacterial population, and the isolate was named "PHARMAQ 001", which corresponds to passage 113.

The bacterial isolate was verified to be *P. salmonis* using a commercial kit "SRS Fluorotest Directo" from Bios Chile, Chile.

PHARMAQ 001 has been deposited with the European Collection of Cell Cultures, Public health England, Culture Collections, Porton Down, Salisbury SP4 OJG, United Kingdom, on 09th October 2014 and was assigned accession number 14100901.

### Example 2: Analysis of PHARMAQ 001

The genomes of PHARMAQ 001 and the virulent starting strain were sequenced and the sequences were compared.

Genetic differences between the two strains, i.e. mutations accumulated by the PHARMAQ 001 strain during its production, were identified.

Because these genetic differences underpin the observed differences in the virulence of PHARMAQ 001 and the starting strain, mutations were identified in PHARMAQ 001 that resulted in a significant change in the amino acid sequence of the encoded protein. Specifically, four significant mutations in PHARMAQ 001 relative to the starting strain were identified. The identity of the four genes was determined, and the genes were found to be *rpoD, ATP-grasp domain protein,* and *FtsH* (all annotated by the IGS Prokaryotic Annotation Pipeline), *and FecR* (GenBank reference: KGB63484.1)

PCR primers were designed to allow the specific region of each of the *rpoD, ATP-grasp domain protein, FtsH,* and *FecR* genes containing the identified mutation to be amplified. In each of the gene sequences shown below, the mutation is shown in bold and underlined, and the primer binding sites are highlighted in grey and underlined.
PHARMAQ 001 *rpoD* gene sequence (Seq. ID No. 1): 
ATGGATCAACAAGAAAAAAGGTCGCAGTTTAAAGAACTCATTGTTCGAGGTAAACAGCAA
GGCTTTTTAACGTTTACAGAGGTAAACGATCATCTTCCGGATGATATGAGCAGCCCGGAA
GAAGTTGAAGAGATCGTTGCAATGATTAGCGACATGGGCATCCCCGTCTATGAAACTGCA
CCCGATCCTGACAGCTTACTCATGAATGAGCATGCCAGCTCTGCCGAAGATGATGCTGAC
GATGCCGTTGCAGCGCTCGATTCAGATGCTGAGTTTGGGCGAACAACCGACCCAGTACGC
ATGTATATGCGCGAGATGGGCAGCGTTGAGCTATTAACGCGCCAAGGTGAAATTGAGCTG
GCTAAACGCATCGAGGAAGGCGTCAAACAAGCCTTTGAGGCAATCGCCCATTACCCACAA
AGCACAGCGATTATTCTTGAAGAATATGCAAGATTTGAAGCCGAAGAAATCCGTTTAGAT
GATATTATCAGTGGCTATATCACCGAAGAAGATGAAGCTCCGACAAGCAACATCGGCTCC
ATGCTTGATGATGCCAATAAAGCCGATGATAATTTTGAAGCCGCCTTGACAGAAGACGAC
AGTACTGATGACGGTGAGGGTGAGGATGACAATGAAGAAATCCCCGTCGATAACACATTG
GATGTTGAGGAAGCGGCAGAGCGTTTTGCCGAGCTAAAAGCTGCCTATGATGCGGTTATA
CAGGTTCAGGAAAAACACGGAATTCATCATAAAAAAACACAACAGCGTTGTGAAGAACTG
TCTAAAGTATTAATGACATTTCGCCTAAAGCCCAATATGATCGATAAAATCACCAACTAC
TTACATGGCTTACTCAGCCAAGTCCGCAAACATGAGCGTCACATCATGGCTTTGTGCATT
AATCAAGCGAAAATGCCCCGCAAGCTATTTATTGATATTTTCCCAGGCAATGAAACCAAT
CTAGAGTGGATAGAGTATCAAATTAAAGCCGAGCAATCTTACTCTGAAGCACTACAGTCC
CTGGCTCCAGAAGTCACTCGTGCACAGAAAAAGCTCATCTCTCTTGAACAAGAATCAAAC
TTTGATGTCACTGCAATTAAAGAAGTCAATCGTAATATTTCTATTGGTGAGGCCAAGGCC
CATCGTGCTAAAAAAGAAATGGTCGAAGCTAACTTGCGTCTGGTCATCTCCATTGCAAAA
AAATACACCAATCGAGGCTTACAATTTCTCGACCTCATTCAAGAAGGCAATATTGGCCTA
ATGAAAGCGGTAGATAAATTCGAATACCGCCGTGGTTATAAATTCTCAACGTATGCAACA
TGGTGGATTCGCCAGGCAATTACCCGCTCAATTGCTGATCAAGCGCGGACAATCCGGATT
CCTGTACATATGATAGAGACAATTAACAAGCTTAACTGCGTCTCACGTCAAATGATCCAA
GAACTCGGCCGTGAAGCGACTCCTGAGGAGCTTTCTGAACGCATGGAGATGCCAGAGCAT
AAAATCCGTAAAATCCTCAAAATCGCTAAAGAGCCAATTTCTATGGAAACACCGATTGGC
GATGACGAAGATTCACACTTAGGTGATTTTATTGAAGATACCACCATGCAACTCCCCGTT
GACTCAACCATGGGTGATGCGTTAAAGCAAGCCACCAGTGATATTCTCGAAAACCTCACG
CCACGTGAAGCAAAAGTCCTTAGAATGCGCTTTGGTATCGATATGAATACTGACCATACG
CTAGAGGAAGTTGGCAAACAATTTGACGTAACTCGCGAGCGTATTCGCCAAATTGAAGCC
AAAGCCTTACGTAAACTGCGCCACCCAACTCGCTCAGAAATTTTGAAGAGTTTTCTTGAC
TCAGAAGAATAA
*PCR Primers:*
   Forward *rpoD* (Seq. ID No. 2): CCAGGCAATTACCCGCTCAA
   Reverse *rpoD* (Seq. ID No. 3): TCGCCAATCGGTGTTTCCAT
PHARMAQ 001 *FecR* gene sequence (Seq. ID No. 4): 
ATGAAAATTAATCATCAGCCTGGCGGCATAATGTTGATAATGAATAATCATGGTGAAGTG
ATGACAAGCTACTCACATATGATGATTTTTTTttCAAATTATGGCGAAAAAGTAAAGATT
GAGAATCAGCGTATCTTAAATGATAATAAACTGCTATTTAGTAATAGAGTGAGTAGGGTG
CGTTATCGGCCTTGTCTTATTATTGATGCAAAAGATGCTTTATCAGTCTGTTCTGGCGTG
TTTCATTAAGTAAAAAATGAATTCGGAGTTGTTGTTGCAAGCTCTCTTAATGTGATGATA
TACGATTATAAATCAATGTCAGATGAGGATATTATTCATATTTTAAAGTCTGTCAAAAAA
CATCCAAAATTATCTTTAATAGAAAGCAAGATACTTTTTTTAAAAGTGGTAATGAAAGGG
ATAAAATGTCGCCATATTGAGTCGTTACTTAAGGTATCCGGAAGTACTGTGTATACGTAT
TGTATGAATATCAAGTCAAAAGCAAATATTTTtAGTTTTAAAGGGCAGTCCGTAATTCAG
GAATTAGAAAAAAGTCAATTTTTTAGTGATATTTCTATAAAGATAAATATAGATTTCTAT
AACATTAATAATGGAGTAAATGAGAAAAATGTTTGCCAAGTATATAGCCTAGCTTAA
*Primers:*
   Forward *FecR* (Seq. ID No. 5): TGGTGAAGTGATGACAAGCTAC
   Reverse *FecR* (Seq. ID No. 6): ACACAGTACTTCCGGATACCTT
PHARMAQ 001 *ATP-grasp domain protein* gene sequence (Seq. ID No. 7): 
ATGATCAGCCTGTGGAAGACCTATCAGGCGCTTAAAACAAAGGGCATTTTAGGCATTAAT
CAGCGTAATGCTGACTTTATTATTCGCTATAATCAGCGCAAATACTACCCTTTAGTCGAT
GATAAAATCATGACAAAAACCCTTGCGATTAAAGATGGTATTGCCGTCCCTAAATTATAT
GCAACCCTTAAAACTGACCATGATACTCACCATCTGGAGCAAATTTTAGCCAATCGAACG
GATTTTGTCATTAAACCGGCCCGTGGTGCTGGCGGTGATGGTATTTTAGTCATTACCAAC
CGCCATGGTGAGCGTTTTCGCAAAGTCAGTGGTGCACTGTTACACTTAGACGATATTCGT
CATCACATTTCTAATATCTTAAGTGGGGTATACAGTCTTGGTGGCCAACGTGATCAGGCC
ATGATTGAATACCGCGTACAATTTGATCCATTATTTAAAAAAATCAGCTATCAAGGTGTG
CCCGATATTCGTATTATTGTCCTAAAAGGCTATCCTGCGATGGCGATGGTGCGTCTACCC
ACTCGGCTCCCTGATGGCAAAGCCAACCTTCATCAAGGTGCAATTGGCGTTGGCATTGAC
TTAACAACAGGCATCACCTTAGAAGGTGTTTGGATGAATGACCCAATTCATGAACATCCG
GATACTGGCTATGCTGTACCAGGCTTACAGATTCCTCACTGGGATCAtTTTTTAAACCTT
GCTGCACGCTGTTACGAGCTTACTCAACTAGGTTATTTAGGTGTGGATATTATCCTTGAT
AAAGACAAAGGCCCACTCATGCTTGAGCTTAATGCGCGTCCTGGTTTAAATATTCAAATT
GCGAATAATAGCGGCTTATTGCATCGATTACGTTTCATTGAGCAACAAAATCAACAACGC
ACAGCCGATGAACGCATTGCTTTCATCAAACATCAGTTCGCAAAAATATAA
*Primers:*
   Forward *ATP-grasp domain protein* (Seq. ID No. 8): GGTGAGCGTTTTCGCAAAGT
   Reverse *ATP-grasp domain protein* (Seq. ID No. 9): TCAAGCATGAGTGGGCCTTT
PHARMAQ 001 *FtsH* gene sequence (Seq. ID No. 10): 
ATGATTAAAAACATCATGCTATGGCTGGTCATTGCTTTGGTGTTGGTGACTGTGTTTAGT
AATTTAGGCCCACGTCAGCAGTCGGTGAATCGGCTAGATTATTCAACATTTGTTAAAGAC
ATCAATAATGGTCAAGTAAAAAGCGTTATCATTGATGGTTTGAATATTAAAGGACAAACC
TCAAGTGGGACGCCATTTGCTACTTATATTCCGTGGAAAGATCCATTTTTAATGGATCAG
ATGCTGGCGAAAAATGTCACAATTGCTGCTAAACCACCTGAGCAGCGGAGCTGGTTATTG
TCTGCATTAATCAGTTGGTTCCCTGGTATTTTATTAATTGCGATTTGGATTTTCTTCTTG
CGGCAGATGCAAGGCGGTGGTGGTGGTAAGGGCATGATGTCCTTTGGTTCCAGTAAGGCA
CGTCTGCTTGGTGAAGATCAAATTAAAGTTAACTTTGCTGATGTTGCTGGCTGTGAAGAG
GCTAAAGAAGAAGTAAAAGAACTGGTCGATTTTCTGCGTGACCCAACCAAATTCCAAAAG
TTAGGCGGCAAAATTCCGCAAGGGGTATTGATAGTTGGCCCACCTGGAACAGGTAAGACG
CTATTAGCTAAAGCCATTGCAGGTGAGGCGAAAGTCCCGTTCTTTTCTATTTCAGGCTCT
GATTTTGTTGAAATGTTCGTCGGTGTCGGTGCATCGCGGGTGCGTGATATGTTTGATCAG
 
GCAAAAAAACGTGCACCGTGTATTATCTTTATCGATGAGATTGATGCAGTGGGCCGTCAC
CGTGGCTCAGGTATGGGCGGTGGTCATGACGAACGTGAGCAGACCTTAAATCAAATGCTG
GTCGAGATGGATGGTTTTGAGGGAACCGAAGGGGTGATTGTCATTGCCGCGACGAATCGT
CCGGATGTATTGGACCCGGCATTATTGCGTCCCGGGCGTTTTGATCGCCAGGTCAGTGTC
GGGCTTCCCGATGTCAAAGGCCGTGAGCAGATTCTAAAAGTGCATATGCGTAAGGTGCCT
TTGGGAGATGATGTTAAAGCGTCATTGATCGCCCGTGGTACGCCTGGGTTCTCAGGAGCG
GATTTGGCGAACTTGGTCAATGAAGCCGCACTCTTTGCCGCGCGTAAAGATAAAACCGTG
GTTGCTATGCGTGAGTTTGATGATGCCAAAGATAAAATTTTGATGGGCACTGAGCGCCGT
TCGATGGCAATGACCGAAGAGCAAAAACGTTTAACCGCCTTTCATGAGGCAGGGCACGCG
ATTGTCGGGTGTTTGGTACCTGATCATGATCCGGTGTATAAAGTCTCGATTGTGCCGCGG
GGTCGTGCCTTAGGTGTGACCATGTATCTGCCTGAAGAGGATAGTTATGGTTATTCACGC
GAGCGCTTGGAGAGCTTAATTTCGAGTATGTATGGCGGACGTATTGCTGAAGCTTTAGTC
TTTGGTGTTGAGAAAGTAACGACTGGGGCATCGAATGACATTGAAAAAGCCTCAGAAGTG
GCGCGCAATATGGTGACAAAGTGGGGGCTGTCTGAGCGCTTAGGGCCGATATTATATGGA
CAAGAAGGCGGTGATCCGTTTGGTTATGGTGCGGGTAAAGGCACGCCGGAATTTTCAGAT
CAAACCTCTGTTGCTATTGATGAGGAAGTACGTCAGATCATTGATCGTAATTATACACGC
GCTGAGAGCATTCTAATCAATAATCGGGATATTCTTGATGCGATGGCGGATGCGTTGATG
GTCTATGAGACGATTGATCGTGACCAAGTGGCTGATCTAATGGCGCGTCGGCCGGTGAAA
GCACCGAAAGATTGGGATCAGCCCTCTGATGAGAGTGGCTCATCAGCATCTGGTGATGAG
TTACAACCTCTTGATGCTAATATCAATACTGATATTAATGAGACTAAGAGCGCTGATCAA
GAGACAGATCAGGGCGCGCCGTCACCAGAAATAAAGGGTAAACCAGCGGATGATCCTACC
TAA
*Primers:*
   Forward *FtsH* (Seq. ID No. 11): TGGTTCCAGTAAGGCACGTC
   Reverse *FtsH* (Seq. ID No. 12): ACAATCACCCCTTCGGTTCC

The mutations in the *rpoD, FecR, ATP-grasp domain protein,* and *FtsH* genes observed in PHARMAQ 001 have been found to be unique by comparison with DNA sequences from other strains of *P. salmonis.* For the purpose of the present disclosure, the mutations found in the *P. salmonis* strain PHARMAQ 001 (shown in bold and underlined in the sequences above) are considered to represent single nucleotide polymorphisms and are located at positions corresponding to:
- residue number 1417 of Seq. ID No. 1 (in the *rpoD* gene);
- residue number 247 of Seq. ID No. 4 (in the *FecR gene*);
- residue number 550 of Seq. ID No. 7 (in *the ATP-grasp domain protein* gene); and,
- residue number 573 of Seq. ID No. 10 (in the *FtsH* gene).

Table 1 shows the occurrence of the specific mutations in the *rpoD, FecR, ATP-grasp domain protein,* and *FtsH* genes observed in PHARMAQ 001 (described in Example 2) in 10 different wild-type virulent strains of *P. salmonis.*

**Table 1**

| **Strain** | **Origin** | **Group** | **Gene** | | | |
|---|---|---|---|---|---|---|
| | | | **rpoD** | **FecR** | ***ATP-gbp*** | ***FtsH*** |
| PHARMAQ 001 | Atlantic salmon, Chile | EM-90 like | X | X | X | X |
| AL 10 005 | Atlantic salmon, Chile | EM-90 like | n | n | n | n |
| AL 20 218 | Atlantic salmon, Chile | EM-90 like | n | n | n | n |
| AL 20 223 | Atlantic salmon, Chile | LF-89 like | n | n | n | n |
| AL 20 220 | Trout, Chile | LF-89 like | n | n | n | n |
| AL 20 471 | Trout, Chile | LF-89 like | n | n | n | n |
| AL 20 222 | Trout, Chile | LF-89 like | n | n | n | n |
| A1-15972 | Atlantic salmon, Chile | EM-90 like | n | n | n | n |
| B1-32597 | Coho salmon, Chile | LF-89 like | n | n | n | n |
| LF-89 | Coho salmon, Chile | LF-89 | n | n | n | n |
| EM-90 | Atlantic salmon, Chile | EM-90 | n | n | n | n |

In Table 1, "X" indicates that the specific mutation found in PHARMAQ 001 (described in Example 2) in each of the *rpoD, ATP-grasp domain protein, FtsH,* or *FecR* genes is present, and "n" indicates that the mutation is not present. As shown in Table 1, none of the strains of *P. salmonis* that were examined were found to possess any of the mutations described in Example 2 in the *rpoD, FecR, ATP-grasp domain protein,* and *FtsH* genes.

Thus, only PHARMAQ 001, and no other strains of *P. salmonis,* possesses the described mutations in the *rpoD, FecR, ATP-grasp domain protein,* and *FtsH* genes.

The presence of these mutations in the *rpoD, FecR, ATP-grasp domain protein,* and *FtsH* genes therefore provides a means of differentiating and distinguishing the PHARMAQ 001 strain from other *Piscirickettsia salmonis* strains.

A method of identifying the PHARMAQ 001 strain involves analysing the DNA sequence of the specific portions of each of the *rpoD, FecR, ATP-grasp domain protein,* and *FtsH* genes containing the identified mutation. The specific portions of the genes may be amplified by polymerase chain reaction (PCR) using specific DNA primers (shown above) followed by DNA sequencing using standard methods. When compared to the sequences of LF-89 (and other wild type strains) the sequences of each of the specific amplified portions of the *rpoD, FecR, ATP-grasp domain protein,* and *FtsH* genes harbors a DNA point mutation which is specific and unique for PHARMAQ 001.

Mutations in the *rpoD, FecR, ATP-grasp domain protein,* and *FtsH* genes were identified due to the fact that they are the four mutations in PHARMAQ 001 which result in a significant change in the amino acid sequence of a protein.

Amino acid sequence alignments between a number of virulent wild-type strains of *P*. *salmonis* and the PHARMAQ 001 attenuated strain for each of the *rpoD, FecR, ATP-grasp domain protein,* and *FtsH* gene products were investigated and are shown below.

The amino acid sequences of *P. salmonis* strains LF-89, EM-90, A1-15972, and B1-32597 were obtained from the published genome sequences of these strains (LF-89, DDBJ/EMBL/GenBank accession no. AMFF00000000.2; EM-90, GenBank accession no.: JRHP00000000.1; A1-15972, GenBank accession no.: JRAV00000000.2; and B1-32597, GenBank accession no.: JRAD00000000.2). The amino acid sequences for the other strains listed below were obtained from the relevant virulent wild-type strain by means of standard PCR and sequencing methods using the PCR primer pairs described above (Seq. ID No.s 2 and 3, 5 and 6, 8 and 9, 11 and 12).
*rpoD* amino acid sequence alignment:

PHARMAQ_001 MDQQEKRSQF KELIVRGKQQ GFLTFTEVND HLPDDMSSPE EVEEIVAMIS DMGIPVYETA
A1-15972 MDQQEKRSQF KELIVRGKQQ GFLTFTEVND HLPDDMSSPE EVEEIVAMIS DMGIPVYETA
B1-32597 MDQQEKKSQF KELIVRGKQQ GFLTFTEVND HLPDDMSSPE EVEEIVAMIS DMGIPVYETA
EM-90 MDQQEKRSQF KELIVRGKQQ GFLTFTEVND HLPDDMSSPE EVEEIVAMIS DMGIPVYETA
LF-89 MDQQEKRSQF KELIVRGKQQ GFLTFTEVND HLPDDMSSPE EVEEIVAMIS DMGIPVYETA
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20222 ---------- ---------- ---------- ---------- ---------- ----------
AL20223 ---------- ---------- ---------- ---------- ---------- ----------
AL20277 ---------- ---------- ---------- ---------- ---------- ----------

61
PHARMAQ_001 PDPDSLLMNE HASSAEDDAD DAVAALDSDA EFGRTTDPVR MYMREMGSVE LLTRQGEIEL
A1-15972 PDPDSLLMNE HASSAEDDAD DAVAALDSDA EFGRTTDPVR MYMREMGSVE LLTRQGEIEL
B1-32597 PDPDSLLMNE HASSAEDDAD DAVAALGSDA EFGRTTDPVR MYMREMGSVE LLTRQGEIEL
EM-90 PDPDSLLMNE HASSAEDDAD DAVAALDSDA EFGRTTDPVR MYMREMGSVE LLTRQGEIEL
LF-89 PDPDSLLMNE HASSAEDDAD DAVAALDSDA EFGRTTDPVR MYMREMGSVE LLTRQGEIEL
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20222 ---------- ---------- ---------- ---------- ---------- ----------
AL20223 ---------- ---------- ---------- ---------- ---------- ----------
AL20277 ---------- ---------- ---------- ---------- ---------- ----------

121
PHARMAQ_001 AKRIEEGVKQ AFEAIAHYPQ STAIILEEYA RFEAEEIRLD DIISGYITEE DEAPTSNIGS
A1-15972 AKRIEEGVKQ AFEAIAHYPQ STAIILEEYA RFEAEEIRLD DIISGYITEE DEAPTSNIGS
B1-32597 AKRIEEGVKQ AFEAIAHYPQ STAIILEEYA RFEAEEIRLD DIISGYITEE DEAPTSNIGS
EM-90 AKRIEEGVKQ AFEAIAHYPQ STAIILEEYA RFEAEEIRLD DIISGYITEE DEAPTSNIGS

LF-89 AKRIEEGVKQ AFEAIAHYPQ STAIILEEYA RFEAEEIRLD DIISGYITEE DEAPTSNIGS
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20222 ---------- ---------- ---------- ---------- ---------- ----------
AL20223 ---------- ---------- ---------- ---------- ---------- ----------
AL20277 ---------- ---------- ---------- ---------- ---------- ----------

181
PHARMAQ_001 MLDDANKADD NFEAALTEDD STDDGEGEDD NEEIPVDNTL DVEEAAERFA ELKAAYDAVI
A1-15972 MLDDANKADD NFEAALTEDD STDDGEGEDD NEEIPVDNTL DVEEAAERFA ELKAAYDAVI
B1-32597 MLHDANKADD NFEAALTEDD STDDAEDEGD NEEIPVDNTL DVEEAAERFA ELKAAYDAVI
EM-90 MLDDANKADD NFEAALTEDD STDDGEGEDD NEEIPVDNTL DVEEAAERFA ELKAAYDAVI
LF-89 MLDDANKADD NFEAALTEDD STDDGEGEDD NEEIPVDNTL DVEEAAERFA ELKAAYDAVI
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20222 ---------- ---------- ---------- ---------- ---------- ----------
AL20223 ---------- ---------- ---------- ---------- ---------- ----------
AL20277 ---------- ---------- ---------- ---------- ---------- ----------

241
PHARMAQ_001 QVQEKHGIHH KKTQQRCEEL SKVLMTFRLK PNMIDKITNY LHGLLSQVRK HERHIMALCI
A1-15972 QVQEKHGIHH KKTQQRCEEL SKVLMTFRLK PNMIDKITNY LHGLLSQVRK HERHIMALCI
B1-32597 QVQEKHGIHH KKTQQRCEEL SKVLMTFRLK PNMIDKITNY LHDLLSQVRK HERHIMALCI
EM-90 QVQEKHGIHH KKTQQRCEEL SKVLMTFRLK PNMIDKITNY LHGLLSQVRK HERHIMALCI
LF-89 QVQEKHGIHH KKTQQRCEEL SKVLMTFRLK PNMIDKITNY LHGLLSQVRK HERHIMALCI
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20222 ---------- ---------- ---------- ---------- ---------- ----------
AL20223 ---------- ---------- ---------- ---------- ---------- ----------
AL20277 ---------- ---------- ---------- ---------- ---------- ----------

301
PHARMAQ_001 NQAKMPRKLF IDIFPGNETN LEWIEYQIKA EQSYSEALQS LAPEVTRAQK KLISLEQESN
A1-15972 NQAKMPRKLF IDIFPGNETN LEWIEYQIKA EQSYSEALQS LAPEVTRAQK KLISLEQESN
B1-32597 NQAKMPRKLF IDIFPGNETN LDWIEYQIKA EQSYSEALQS LAPEVTRAQK KLISLEQESN
EM-90 NQAKMPRKLF IDIFPGNETN LEWIEYQIKA EQSYSEALQS LAPEVTRAQK KLISLEQESN
LF-89 NQAKMPRKLF IDIFPGNETN LEWIEYQIKA EQSYSEALQS LAPEVTRAQK KLISLEQESN
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20222 ---------- ---------- ---------- ---------- ---------- ----------
AL20223 ---------- ---------- ---------- ---------- ---------- ----------
AL20277 ---------- ---------- ---------- ---------- ---------- ----------

361
PHARMAQ_001 FDVTAIKEVN RNISIGEAKA HRAKKEMVEA NLRLVISIAK KYTNRGLQFL DLIQEGNIGL
A1-15972 FDVTAIKEVN RNISIGEAKA HRAKKEMVEA NLRLVISIAK KYTNRGLQFL DLIQEGNIGL
B1-32597 FDVTAIKEVN RNISIGEAKA HRAKKEMVEA NLRLVISIAK KYTNRGLQFL DLIQEGNIGL
EM-90 FDVTAIKEVN RNISIGEAKA HRAKKEMVEA NLRLVISIAK KYTNRGLQFL DLIQEGNIGL
LF-89 FDVTAIKEVN RNISIGEAKA HRAKKEMVEA NLRLVISIAK KYTNRGLQFL DLIQEGNIGL
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20222 ---------- ---------- ---------- ---------- ---------- ----------
AL20223 ---------- ---------- ---------- ---------- ---------- ----------
AL20277 ---------- ---------- ---------- ---------- ---------- ----------

421
                                                                    *
PHARMAQ_001 MKAVDKFEYR RGYKFSTYAT WWIRQAITRS IADQARTIRI PVHMIETINK LNCVSRQMIQ
A1-15972    MKAVDKFEYR RGYKFSTYAT WWIRQAITRS IADQARTIRI PVHMIETINK LNRVSRQMIQ
B1-32597    MKAVDKFEYR RGYKFSTYAT WWIRQAITRS IADQARTIRI PVHMIETINK LNRVSRQMIQ
EM-90       MKAVDKFEYR RGYKFSTYAT WWIRQAITRS IADQARTIRI PVHMIETINK LNRVSRQMIQ
LF-89       MKAVDKFEYR RGYKFSTYAT WWIRQAITRS IADQARTIRI PVHMIETINK LNRVSRQMIQ
AL10016     ---------- ---------- ---------- ---------- -VHMIETINK LNRVSRQMIQ
AL10008     ---------- ---------- ---------- ---------- -VHMIETINK LNRVSRQMIQ
AL20218     ---------- ---------- ---------- ---------- -VHMIETINK LNRVSRQMIQ
AL20219     ---------- ---------- ---------- ---------- -VHMIETINK LNRVSRQMIQ
AL20222     ---------- ---------- ---------- ---------- -VHMIETINK LNRVSRQMIQ
AL20223     ---------- ---------- ---------- ---------- -VHMIETINK LNRVSRQMIQ
AL20277     ---------- ---------- ---------- ---------- -VHMIETINK LNRVSRQMIQ

481
PHARMAQ_001 ELGREATPEE LSERMEMPEH KIRKILKIAK EPISMETPIG DDEDSHLGDF IEDTTMQLPV
A1-15972    ELGREATPEE LSERMEMPEH KIRKILKIAK EPISMETPIG DDEDSHLGDF IEDTTMQLPV
B1-32597    ELGREATPEE LSERMEMPEH KIRKILKIAK EPISMETPIG DDEDSHLGDF IEDTTMQLPV
EM-90       ELGREATPEE LSERMEMPEH KIRKILKIAK EPISMETPIG DDEDSHLGDF IEDTTMQLPV
LF-89       ELGREATPEE LSERMEMPEH KIRKILKIAK EPISMETPIG DDEDSHLGDF IEDTTMQLPV
AL10016     ELGREATPEE LSERMEMPEH KIRK------ ---------- ---------- ----------
AL10008     ELGREATPEE LSERMEMPEH KIRK------ ---------- ---------- ----------
AL20218     ELGREATPEE LSERMEMPEH KIRK------ ---------- ---------- ----------
AL20219     ELGREATPEE LSERMEMPEH KIRK------ ---------- ---------- ----------
AL20222     ELGREATPEE LSERMEMPEH KIRK------ ---------- ---------- ----------
AL20223     ELGREATPEE LSERMEMPEH KIRK------ ---------- ---------- ----------
AL20277     ELGREATPEE LSERMEMPEH KIRK------ ---------- ---------- ----------

541
PHARMAQ_001 DSTMGDALKQ ATSDILENLT PREAKVLRMR FGIDMNTDHT LEEVGKQFDV TRERIRQIEA
A1-15972    DSTMGDALKQ ATSDILENLT PREAKVLRMR FGIDMNTDHT LEEVGKQFDV TRERIRQIEA
B1-32597    DSTMGDALKQ ATSDILENLT PREAKVLRMR FGIDMNTDHT LEEVGKQFDV TRERIRQIEA
EM-90       DSTMGDALKQ ATSDILENLT PREAKVLRMR FGIDMNTDHT LEEVGKQFDV TRERIRQIEA
LF-89       DSTMGDALKQ ATSDILENLT PREAKVLRMR FGIDMNTDHT LEEVGKQFDV TRERIRQIEA
AL10016     ---------- ---------- ---------- ---------- ---------- ----------
AL10008     ---------- ---------- ---------- ---------- ---------- ----------
AL20218     ---------- ---------- ---------- ---------- ---------- ----------
AL20219     ---------- ---------- ---------- ---------- ---------- ----------
AL20222     ---------- ---------- ---------- ---------- ---------- ----------
AL20223     ---------- ---------- ---------- ---------- ---------- ----------
AL20277     ---------- ---------- ---------- ---------- ---------- ----------

601
PHARMAQ_001 KALRKLRHPT RSEILKSFLD SEE* (Seq. ID No. 13)
A1-15972    KALRKLRHPT RSEILKSFLD SEE* (Seq. ID No. 14)
B1-32597    KALRKLRHPT RSEILKSFLD SEE* (Seq. ID No. 15)
EM-90       KALRKLRHPT RSEILKSFLD SEE* (Seq. ID No. 16)
LF-89       KALRKLRHPT RSEILKSFLD SEE* (Seq. ID No. 17)
AL10016     ---------- ---------- ---- (Seq. ID No. 18)
AL10008     ---------- ---------- ---- (Seq. ID No. 19)
AL20218     ---------- ---------- ---- (Seq. ID No. 20)
AL20219     ---------- ---------- ---- (Seq. ID No. 21)
AL20222     ---------- ---------- ---- (Seq. ID No. 22)
AL20223     ---------- ---------- ---- (Seq. ID No. 23)
AL20277     ---------- ---------- ---- (Seq. ID No. 24)

The *rpoD* amino acid sequence alignment reveals that there are natural polymorphisms of the *rpoD* gene product between wild-type strains. However, all of the wild-type strains are virulent and therefore none of the differences between the wild-type sequences can be considered to affect virulence. PHARMAQ 001 has an arginine to cysteine mutation at position 473 of the amino acid sequence of the *rpoD* gene product and this mutation is not seen in any of the wild-type strains investigated, such as A1-15972, B1-32597, EM-90, and/or LF-89. The protein sequence of the *rpoD* gene product in PHARMAQ 001 is otherwise identical to that of wild-type strains including A1-15972, EM-90, and LF-89. The sequences of 11 wild-type strains were examined in the region of amino acid residues 462-504 of the *rpoD* gene product. All of the sequences were found to be identical in this region, but different to that of PHARMAQ 001.
*FecR* amino acid sequence alignment:

PHARMAQ_001 MKINHQPGGI MLIMNNHGEV MTSYSHMMIF FSNYGEKVKI ENQRILNDNK LLFSNRVSRV
LF-89 MKINHQPGGI MLIMNNHGEV MTSYSHMMSF FSNYGEKVKI ENQRILNDNK LLFSNRVSRV
EM-90 MKINHQPGGI MLIMNNHGEV MTSYSHMMSF FSNYGEKVKI ENQRILNDNK LLFSNRVSRV
B1-32597 MKINHQPGGI MLIMNNHGEV MTSYSHMMSF FSNYGEKVKI ENQRILNDNK LLFSNRVSEV
A1-15972 MKINHQPGGI MLIMNNHGEV MTSYSHMMIF FSNYGEKVKI ENQRILNDNK LLFSNRVSRV
AL10016 ---------- ---------- ---------- -------?KI ENQRILNDNK LLFSNRVSRV
AL10008 ---------- ---------- ---------- -------?KI ENQRILNDNK LLFSNRVSRV
AL20218 ---------- ---------- ---------- -------?KI ENQRILNDNK LLFSNRVSRV
AL20219 ---------- ---------- ---------- -------?KI ENQRILNDNK LLFSNRVSRV
AL20220 ---------- ---------- ---------- -------?KI ENQRILNDNK LLFSNRVSEV
AL20223 ---------- ---------- ---------- -------?KI ENQRILNDNK LLFSNRVSEV
AL20470 ---------- ---------- ---------- -------?KI ENQRILNDNK LLFSNRVSEV
AL20471 ---------- ---------- ---------- -------?KI ENQRILNDNK LLFSNRVSEV
AL20455 ---------- ---------- ---------- -------?KI ENQRILNDNK LLFSNRVSEV

61
*
PHARMAQ_001 RYRPCLIIDA KDALSVCSGV FH*------- ---------- ---------- ----------
LF-89 RYRPCLIIDA KDALSVCSGV FHQVKNEFGV VVASSLNVMI YDYKSMSDED IIHILKSVKK
EM-90 RYRPCLIIDA KDALSVCSGV FHQVKNEFGV VVASSLNVMI YDYKSMSDED IIHILKSVKK
B1-32597 RYRPCLIIDA KDAFSVCSGV FHQVKNEFGV VVANSLNVMI YDYKSMSDED IIHILKSVKK
A1-15972 RYRPCLIIDA KDALSVCSGV FHQVKNEFGV VVASSLNVMI YDYKSMSDED IIHILKSVKK
AL10016 RYRPCLIIDA KDALSVCSGV FHQVKNEFGV VVASSLNVMI YDYKSMSDED IIHILKSVKK
AL10008 RYRPCLIIDA KDALSVCSGV FHQVKNEFGV VVASSLNVMI YDYKSMSDED IIHILKSVKK
AL20218 RYRPCLIIDA KDALSVCSGV FHQVKNEFGV VVASSLNVMI YDYKSMSDED IIHILKSVKK
AL20219 RYRPCLIIDA KDALSVCSGV FHQVKNEFGV VVASSLNVMI YDYKSMSDED IIHILKSVKK
AL20220 RYRPCLIIDA KDAFSVCSGV FHQVKNEFGV VVANSLNVMI YDYKSMSDED IIHILKSVKK
AL20223 RYRPCLIIDA KDAFSVCSGV FHQVKNEFGV VVANSLNVMI YDYKSMSDED IIHILKSVKK
AL20470 RYRPCLIIDA KDAFSVCSGV FHQVKNEFGV VVANSLNVMI YDYKSMSDED IIHILKSVKK
AL20471 RYRPCLIIDA KDAFSVCSGV FHQVKNEFGV VVANSLNVMI YDYKSMSDED IIHILKSVKK
AL20455 RYRPCLIIDA KDAFSVCSGV FHQVKNEFGV VVANSLNVMI YDYKSMSDED IIHILKSVKK

121
PHARMAQ_001 ---------- ---------- ---------- ---------- ---------- ----------
LF-89 HPKLSLIESK ILFLKVVMKG IKCRHIESLL KVSGSTVYTY CMNIKSKANI FSFKGQSVIQ
EM-90 HPKLSLIESK ILFLKVVMKG IKCRHIESLL KVSGSTVYTY CMNIKSKANI FSFKGQSVIQ
B1-32597 HPKLSLIESK ILFLKVVMKG IKCRHIESLL KVSGSTVYTY CMNIKSKANI FSFKGQSVIQ
A1-15972 HPKLSLIESK ILFLKVVMKG IKCRHIESLL KVSGSTVYTY CMNIKSKANI FSFKGQSVIQ
AL10016 HPKLSLIESK ILFLKVV?-- ---------- ---------- ---------- ----------
AL10008 HPKLSLIESK ILFLKVV?-- ---------- ---------- ---------- ----------
AL20218 HPKLSLIESK ILFLKVV?-- ---------- ---------- ---------- ----------
AL20219 HPKLSLIESK ILFLKVV?-- ---------- ---------- ---------- ----------
AL20220 HPKLSLIESK ILFLKVV?-- ---------- ---------- ---------- ----------
AL20223 HPKLSLIESK ILFLKVV?-- ---------- ---------- ---------- ----------
AL20470 HPKLSLIESK ILFLKVV?-- ---------- ---------- ---------- ----------
AL20471 HPKLSLIESK ILFLKVV?-- ---------- ---------- ---------- ----------
AL20455 HPKLSLIESK ILFLKVV?-- ---------- ---------- ---------- ----------

181
PHARMAQ_001 ---------- ---------- ---------- --------- (Seq. ID No. 25)
LF-89 ELEKSQFFSD ISIKINIDFY NINNGVNEKN VCQVYSLA* (Seq. ID No. 26)
EM-90 ELEKSQFFSD ISIKINIDFY NINNGVNEKN VCQVYSLA* (Seq. ID No. 27)
B1-32597 ELEKSQFFSD IAMKINIDFY SINNEANEKN VCQVYSLA* (Seq. ID No. 28)
A1-15972 ELEKSQFFSD ISIKINIDFY NINNGVNEKN VCQVYSLA* (Seq. ID No. 29)
AL10016 ---------- ---------- ---------- --------- (Seq. ID No. 30)
AL10008 ---------- ---------- ---------- --------- (Seq. ID No. 31)
AL20218 ---------- ---------- ---------- --------- (Seq. ID No. 32)
AL20219 ---------- ---------- ---------- --------- (Seq. ID No. 33)
AL20220 ---------- ---------- ---------- --------- (Seq. ID No. 34)
AL20223 ---------- ---------- ---------- --------- (Seq. ID No. 35)
AL20470 ---------- ---------- ---------- --------- (Seq. ID No. 36)
AL20471 ---------- ---------- ---------- --------- (Seq. ID No. 37)
AL20455 ---------- ---------- ---------- --------- (Seq. ID No. 38)

The *FecR* amino acid sequence alignment reveals that there are natural polymorphisms of the *FecR* gene product between wild-type strains. However, all of the wild-type strains are virulent and therefore none of the differences between the wild-type sequences can be considered to affect virulence. PHARMAQ 001 has a premature stop codon introduced at position 83 of the amino acid, and this mutation is not seen in any of the wild-type strains investigated, such as A1-15972, B1-32597, EM-90, and/or LF-89. The protein sequence of the *FecR* gene product in PHARMAQ 001 is otherwise identical to that of wild-type strains including A1-15972, EM-90, and LF-89. The sequences of 13 wild-type strains were examined in the region of amino acid residues 39-137 of the *FecR* gene product. None of the wild-type sequences were found to be prematurely truncated, whereas in contrast, the *FecR* gene product of PHARMAQ 001 is terminated by a stop codon in position 83.
*ATP-grasp domain protein* amino acid sequence alignment:

PHARMAQ001 MISLWKTYQA LKTKGILGIN QRNADFIIRY NQRKYYPLVD DKIMTKTLAI KDGIAVPKLY
LF-89 MISLWKTYQA LKTKGILGIN QRNADFIIRY NQRKYYPLVD DKIMTKTLAI KDGIAVPKLY
EM-90 MISLWKTYQA LKTKGILGIN QRNADFIIRY NQRKYYPLVD DKIMTKTLAI KDGIAVPKLY
B1-32597 MISLWKTYQA LKTKGILGIN QRNADFIIRY NQRKYYPLVD DKIMTKTLAI KDGIAVPKLY
A1-15972 MISLWKTYQA LKTKGILGIN QRNADFIIRY NQRKYYPLVD DKIMTKTLAI KDGIAVPKLY
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20220 ---------- ---------- ---------- ---------- ---------- ----------
AL20223 ---------- ---------- ---------- ---------- ---------- ----------
AL20470 ---------- ---------- ---------- ---------- ---------- ----------
AL20471 ---------- ---------- ---------- ---------- ---------- ----------
AL20455 ---------- ---------- ---------- ---------- ---------- ----------

61
PHARMAQ001 ATLKTDHDTH HLEQILANRT DFVIKPARGA GGDGILVITN RHGERFRKVS GALLHLDDIR
LF-89 ATLKTDHDTH HLEQILANRT DFVIKPARGA GGDGILVITN RHGERFRKVS GALLHLDDIR
EM-90 ATLKTDHDTH HLEQILANRT DFVIKPARGA GGDGILVITN RHGERFRKVS GALLHLDDIR
B1-32597 ATLKTDHDTH HLEQILANRT DFVIKPARGA GGDGILVITN RHGERFRKVS GALLHLDDIR
A1-15972 ATLKTDHDTH HLEQILANRT DFVIKPARGA GGDGILVITN RHGERFRKVS GALLHLDDIR
AL10016 ---------- ---------- ---------- ---------- ---------- ------?DIR
AL10008 ---------- ---------- ---------- ---------- ---------- ------?DIR

AL20218     ---------- ---------- ---------- ---------- ---------- ------?DIR
AL20219     ---------- ---------- ---------- ---------- ---------- ------?DIR
AL20220     ---------- ---------- ---------- ---------- ---------- ------?DIR
AL20223     ---------- ---------- ---------- ---------- ---------- ------?DIR
AL20470     ---------- ---------- ---------- ---------- ---------- ------?DIR
AL20471     ---------- ---------- ---------- ---------- ---------- ------?DIR
AL20455     ---------- ---------- ---------- ---------- ---------- ------?DIR

121
PHARMAQ001  HHISNILSGV YSLGGQRDQA MIEYRVQFDP LFKKISYQGV PDIRIIVLKG YPAMAMVRLP
LF-89       HHISNILSGV YSLGGQRDQA MIEYRVQFDP LFKKISYQGV PDIRIIVLKG YPAMAMVRLP
EM-90       HHISNILSGV YSLGGQRDQA MIEYRVQFDP LFKKISYQGV PDIRIIVLKG YPAMAMVRLP
B1-32597    HHISNILSGV YSLGGQRDQA MIEYRVQFDP LFKKISYQGV PDIRIIVLKG YPAMAMVRLP
A1-15972    HHISNILSGV YSLGGQRDQA MIEYRVQFDP LFKKISYQGV PDIRIIVLKG YPAMAMVRLP
AL10016     HHISNILSGV YSLGGQRDQA MIEYRVQFDP LFKKISYQGV PDIRIIVLKG YPAMAMVRLP
AL10008     HHISNILSGV YSLGGQRDQA MIEYRVQFDP LFKKISYQGV PDIRIIVLKG YPAMAMVRLP
AL20218     HHISNILSGV YSLGGQRDQA MIEYRVQFDP LFKKISYQGV PDIRIIVLKG YPAMAMVRLP
AL20219     HHISNILSGV YSLGGQRDQA MIEYRVQFDP LFKKISYQGV PDIRIIVLKG YPAMAMVRLP
AL20220     HHISNILSGV YSLGGQRDQA MIEYRVQFDP LFKKISYQGV PDIRIIVLKG YPAMAMVRLP
AL20223     HHISNILSGV YSLGGQRDQA MIEYRVQFDP LFKKISYQGV PDIRIIVLKG YPAMAMVRLP
AL20470     HHISNILSGV YSLGGQRDQA MIEYRVQFDP LFKKISYQGV PDIRIIVLKG YPAMAMVRLP
AL20471     HHISNILSGV YSLGGQRDQA MIEYRVQFDP LFKKISYQGV PDIRIIVLKG YPAMAMVRLP
AL20455     HHISNILSGV YSLGGQRDQA MIEYRVQFDP LFKKISYQGV PDIRIIVLKG YPAMAMVRLP

181            *
PHARMAQ001  TRLPDGKANL HQGAIGVGID LTTGITLEGV WMNDPIHEHP DTGYAVPGLQ IPHWDHFLNL
LF-89       TRLSDGKANL HQGAIGVGID LTTGITLEGV WMNDPIHEHP DTGYAVPGLQ IPHWDHFLNL
EM-90       TRLSDGKANL HQGAIGVGID LTTGITLEGV WMNDPIHEHP DTGYAVPGLQ IPHWDHFLNL
B1-32597    TRLSDGKANL HQGAIGVGID LTTGITLEGV WMNDPIHEHP DTGYAVPGLQ IPHWDHFLNL
A1-15972    TRLSDGKANL HQGAIGVGID LTTGITLEGV WMNDPIHEHP DTGYAVPGLQ IPHWDHFLNL
AL10016     TRLSDGKANL HQGAIGVGID LTTGITLEGV WMNDPIHEHP DTGYAVPGLQ IPHWDHFLNL
AL10008     TRLSDGKANL HQGAIGVGID LTTGITLEGV WMNDPIHEHP DTGYAVPGLQ IPHWDHFLNL
AL20218     TRLSDGKANL HQGAIGVGID LTTGITLEGV WMNDPIHEHP DTGYAVPGLQ IPHWDHFLNL
AL20219     TRLSDGKANL HQGAIGVGID LTTGITLEGV WMNDPIHEHP DTGYAVPGLQ IPHWDHFLNL
AL20220     TRLSDGKANL HQGAIGVGID LTTGITLEGV WMNDPIHEHP DTGYAVPGLQ IPHWDHFLNL
AL20223     TRLSDGKANL HQGAIGVGID LTTGITLEGV WMNDPIHEHP DTGYAVPGLQ IPHWDHFLNL
AL20470     TRLSDGKANL HQGAIGVGID LTTGITLEGV WMNDPIHEHP DTGYAVPGLQ IPHWDHFLNL
AL20471     TRLSDGKANL HQGAIGVGID LTTGITLEGV WMNDPIHEHP DTGYAVPGLQ IPHWDHFLNL
AL20455     TRLSDGKANL HQGAIGVGID LTTGITLEGV WMNDPIHEHP DTGYAVPGLQ IPHWDHFLNL

241
PHARMAQ001  AARCYELTQL GYLGVDIILD KDKGPLMLEL NARPGLNIQI ANNSGLLHRL RFIEQQNQQR
LF-89       AARCYELTQL GYLGVDIILD KDKGPLMLEL NARPGLNIQI ANNSGLLHRL RFIEQQNQQR
EM-90       AARCYELTQL GYLGVDIILD KDKGPLMLEL NARPGLNIQI ANNSGLLHRL RFIEQQNQQR
B1-32597    AARCYELTQL GYLGVDIILD KDKGPLMLEL NARPGLNIQI ANNSGLLHRL RFIEQQNQQR
A1-15972    AARCYELTQL GYLGVDIILD KDKGPLMLEL NARPGLNIQI ANNSGLLHRL RFIEQQNQQR
AL10016     AARCYELTQL G?-------- ---------- ---------- ---------- ----------
AL10008     AARCYELTQL G?-------- ---------- ---------- ---------- ----------
AL20218     AARCYELTQL G?-------- ---------- ---------- ---------- ----------
AL20219     AARCYELTQL G?-------- ---------- ---------- ---------- ----------
AL20220     AARCYELTQL G?-------- ---------- ---------- ---------- ----------
AL20223     AARCYELTQL G?-------- ---------- ---------- ---------- ----------
AL20470     AARCYELTQL G?-------- ---------- ---------- ---------- ----------
AL20471     AARCYELTQL G?-------- ---------- ---------- ---------- ----------
AL20455     AARCYELTQL G?-------- ---------- ---------- ---------- ----------

301
PHARMAQ001  TADERIAFIK HQFAKI* (Seq. ID No. 39)
LF-89       TADERIAFIK HQFAKI* (Seq. ID No. 40)
EM-90       TADERIAFIK HQFAKI* (Seq. ID No. 41)
B1-32597    TADERIAFIK HQFAKI* (Seq. ID No. 42)
A1-15972    TADERIAFIK HQFAKI* (Seq. ID No. 43)
AL10016     ---------- ------- (Seq. ID No. 44)
AL10008     ---------- ------- (Seq. ID No. 45)
AL20218     ---------- ------- (Seq. ID No. 46)

AL20219    ---------- ------- (Seq. ID No. 47)
AL20220    ---------- ------- (Seq. ID No. 48)
AL20223    ---------- ------- (Seq. ID No. 49)
AL20470    ---------- ------- (Seq. ID No. 50)
AL20471    ---------- ------- (Seq. ID No. 51)
AL20455    ---------- ------- (Seq. ID No. 52)

All of the wild-type sequences of the *ATP-grasp domain protein* gene product that were investigated were found to be identical. However, PHARMAQ 001 has a serine to proline mutation at position 184 of the amino acid sequence of the *ATP-grasp domain protein* gene product which is not seen in the wild-type sequence. The sequences of 13 wild-type strains were examined in the region of amino acid residues 118-251 of the *ATP-grasp domain protein* gene product. All of the sequences were found to be identical in this region, but different to that of PHARMAQ 001.
*FtsH* amino acid sequence alignment:

PHARMAQ001 MIKNIMLWLV IALVLVTVFS NLGPRQQSVN RLDYSTFVKD INNGQVKSVI IDGLNIKGQT
LF-89 MIKNIMLWLV IALVLVTVFS NLGPRQQSVN RLDYSTFVKD INNGQVKSVI IDGLNIKGQT
EM-90 MIKNIMLWLV IALVLVTVFS NLGPRQQSVN RLDYSTFVKD INNGQVKSVI IDGLNIKGQT
B1_32597 MIKNIMLWLV IALVLVTVFS NLGPRQQSVN RLDYSTFVKD INNGQVKSVI IDGLNIKGQT
A1_15972 MIKNIMLWLV IALVLVTVFS NLGPRQQSVN RLDYSTFVKD INNGQVKSVI IDGLNIKGQT
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20220 ---------- ---------- ---------- ---------- ---------- ----------
AL20223 ---------- ---------- ---------- ---------- ---------- ----------
AL20470 ---------- ---------- ---------- ---------- ---------- ----------
AL20471 ---------- ---------- ---------- ---------- ---------- ----------
AL20455 ---------- ---------- ---------- ---------- ---------- ----------

61
PHARMAQ001 SSGTPFATYI PWKDPFLMDQ MLAKNVTIAA KPPEQRSWLL SALISWFPGI LLIAIWIFFL
LF-89 SSGTPFATYI PWKDPFLMDQ MLAKNVTIAA KPPEQRSWLL SALISWFPGI LLIAIWIFFL
EM-90 SSGTPFATYI PWKDPFLMDQ MLAKNVTIAA KPPEQRSWLL SALISWFPGI LLIAIWIFFL
B1_32597 SSGTPFATYI PWKDPFLMDQ MLSKNVTIAA KPPEQRSWLL SALISWFPGI LLIAIWIFFL
A1_15972 SSGTPFATYI PWKDPFLMDQ MLAKNVTIAA KPPEQRSWLL SALISWFPGI LLIAIWIFFL
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20220 ---------- ---------- ---------- ---------- ---------- ----------
AL20223 ---------- ---------- ---------- ---------- ---------- ----------
AL20470 ---------- ---------- ---------- ---------- ---------- ----------
AL20471 ---------- ---------- ---------- ---------- ---------- ----------
AL20455 ---------- ---------- ---------- ---------- ---------- ----------

121
PHARMAQ001 RQMQGGGGGK GMMSFGSSKA RLLGEDQIKV NFADVAGCEE AKEEVKELVD FLRDPTKFQK
LF-89 RQMQGGGGGK GMMSFGSSKA RLLGEDQIKV NFADVAGCEE AKEEVKELVD FLRDPTKFQK
EM-90 RQMQGGGGGK GMMSFGSSKA RLLGEDQIKV NFADVAGCEE AKEEVKELVD FLRDPTKFQK
B1_32597 RQMQGGGGGK GMMSFGSSKA RLLGEDQIKV NFADVAGCEE AKEEVKELVD FLRDPTKFQK
A1_15972 RQMQGGGGGK GMMSFGSSKA RLLGEDQIKV NFADVAGCEE AKEEVKELVD FLRDPTKFQK
AL10016 ---------- ---------- ---------- -FADVAGCEE AKEEVKELVD FLRDPTKFQK
AL10008 ---------- ---------- ---------- -FADVAGCEE AKEEVKELVD FLRDPTKFQK
AL20218 ---------- ---------- ---------- -FADVAGCEE AKEEVKELVD FLRDPTKFQK
AL20219 ---------- ---------- ---------- -FADVAGCEE AKEEVKELVD FLRDPTKFQK

AL20220      ---------- ---------- ---------- -FADVAGCEE AKEEVKELVD FLRDPTKFQK
AL20223      ---------- ---------- ---------- -FADVAGCEE AKEEVKELVD FLRDPTKFQK
AL20470      ---------- ---------- ---------- -FADVAGCEE AKEEVKELVD FLRDPTKFQK
AL20471      ---------- ---------- ---------- -FADVAGCEE AKEEVKELVD FLRDPTKFQK
AL20455      ---------- ---------- ---------- -FADVAGCEE AKEEVKELVD FLRDPTKFQK

181                     *
PHARMAQ001   LGGKIPQGVL IVGPPGTGKT LLAKAIAGEA KVPFFSISGS DFVEMFVGVG ASRVRDMFDQ
LF-89        LGGKIPQGVL MVGPPGTGKT LLAKAIAGEA KVPFFSISGS DFVEMFVGVG ASRVRDMFDQ
EM-90        LGGKIPQGVL MVGPPGTGKT LLAKAIAGEA KVPFFSISGS DFVEMFVGVG ASRVRDMFDQ
B1_32597     LGGKIPQGVL MVGPPGTGKT LLAKAIAGEA KVPFFSISGS DFVEMFVGVG ASRVRDMFDQ
A1_15972     LGGKIPQGVL MVGPPGTGKT LLAKAIAGEA KVPFFSISGS DFVEMFVGVG ASRVRDMFDQ
AL10016      LGGKIPQGVL MVGPPGTGKT LLAKAIAGEA KVPFFSISGS DFVEMFVGVG ASRVRDMFDQ
AL10008      LGGKIPQGVL MVGPPGTGKT LLAKAIAGEA KVPFFSISGS DFVEMFVGVG ASRVRDMFDQ
AL20218      LGGKIPQGVL MVGPPGTGKT LLAKAIAGEA KVPFFSISGS DFVEMFVGVG ASRVRDMFDQ
AL20219      LGGKIPQGVL MVGPPGTGKT LLAKAIAGEA KVPFFSISGS DFVEMFVGVG ASRVRDMFDQ
AL20220      LGGKIPQGVL MVGPPGTGKT LLAKAIAGEA KVPFFSISGS DFVEMFVGVG ASRVRDMFDQ
AL20223      LGGKIPQGVL MVGPPGTGKT LLAKAIAGEA KVPFFSISGS DFVEMFVGVG ASRVRDMFDQ
AL20470      LGGKIPQGVL MVGPPGTGKT LLAKAIAGEA KVPFFSISGS DFVEMFVGVG ASRVRDMFDQ
AL20471      LGGKIPQGVL MVGPPGTGKT LLAKAIAGEA KVPFFSISGS DFVEMFVGVG ASRVRDMFDQ
AL20455      LGGKIPQGVL MVGPPGTGKT LLAKAIAGEA KVPFFSISGS DFVEMFVGVG ASRVRDMFDQ

241
PHARMAQ001   AKKRAPCIIF IDEIDAVGRH RGSGMGGGHD EREQTLNQML VEMDGFEGTE GVIVIAATNR
LF-89        AKKRAPCIIF IDEIDAVGRH RGSGMGGGHD EREQTLNQML VEMDGFEGTE GVIVIAATNR
EM-90        AKKRAPCIIF IDEIDAVGRH RGSGMGGGHD EREQTLNQML VEMDGFEGTE GVIVIAATNR
B1_32597     AKKRAPCIIF IDEIDAVGRH RGSGMGGGHD EREQTLNQML VEMDGFEGTE GVIVIAATNR
A1_15972     AKKRAPCIIF IDEIDAVGRH RGSGMGGGHD EREQTLNQML VEMDGFEGTE GVIVIAATNR
AL10016      AKKRAPCIIF IDEIDAVGRH RGSGMGGGHD EREQ?----- ---------- ----------
AL10008      AKKRAPCIIF IDEIDAVGRH RGSGMGGGHD EREQ?----- ---------- ----------
AL20218      AKKRAPCIIF IDEIDAVGRH RGSGMGGGHD EREQ?----- ---------- ----------
AL20219      AKKRAPCIIF IDEIDAVGRH RGSGMGGGHD EREQ?----- ---------- ----------
AL20220      AKKRAPCIIF IDEIDAVGRH RGSGMGGGHD EREQ?----- ---------- ----------
AL20223      AKKRAPCIIF IDEIDAVGRH RGSGMGGGHD EREQ?----- ---------- ----------
AL20470      AKKRAPCIIF IDEIDAVGRH RGSGMGGGHD EREQ?----- ---------- ----------
AL20471      AKKRAPCIIF IDEIDAVGRH RGSGMGGGHD EREQ?----- ---------- ----------
AL20455      AKKRAPCIIF IDEIDAVGRH RGSGMGGGHD EREQ?----- ---------- ----------

301
PHARMAQ001   PDVLDPALLR PGRFDRQVSV GLPDVKGREQ ILKVHMRKVP LGDDVKASLI ARGTPGFSGA
LF-89        PDVLDPALLR PGRFDRQVSV GLPDVKGREQ ILKVHMRKVP LGDDVKASLI ARGTPGFSGA
EM-90        PDVLDPALLR PGRFDRQVSV GLPDVKGREQ ILKVHMRKVP LGDDVKASLI ARGTPGFSGA
B1_32597     PDVLDPALLR PGRFDRQVSV GLPDVKGREQ ILKVHMRKVP LGDDVKASLI ARGTPGFSGA
A1_15972     PDVLDPALLR PGRFDRQVSV GLPDVKGREQ ILKVHMRKVP LGDDVKASLI ARGTPGFSGA
AL10016      ---------- ---------- ---------- ---------- ---------- ----------
AL10008      ---------- ---------- ---------- ---------- ---------- ----------
AL20218      ---------- ---------- ---------- ---------- ---------- ----------
AL20219      ---------- ---------- ---------- ---------- ---------- ----------
AL20220      ---------- ---------- ---------- ---------- ---------- ----------
AL20223      ---------- ---------- ---------- ---------- ---------- ----------
AL20470      ---------- ---------- ---------- ---------- ---------- ----------
AL20471      ---------- ---------- ---------- ---------- ---------- ----------
AL20455      ---------- ---------- ---------- ---------- ---------- ----------

361
PHARMAQ001   DLANLVNEAA LFAARKDKTV VAMREFDDAK DKILMGTERR SMAMTEEQKR LTAFHEAGHA
LF-89        DLANLVNEAA LFAARKDKTV VAMREFDDAK DKILMGTERR SMAMTEEQKR LTAFHEAGHA
EM-90        DLANLVNEAA LFAARKDKTV VAMREFDDAK DKILMGTERR SMAMTEEQKR LTAFHEAGHA
B1_32597     DLANLVNEAA LFAARKDKTV VAMREFDDAK DKILMGTERR SMAMTEEQKR LTAFHEAGHA
A1_15972     DLANLVNEAA LFAARKDKTV VAMREFDDAK DKILMGTERR SMAMTEEQKR LTAFHEAGHA
AL10016      ---------- ---------- ---------- ---------- ---------- ----------
AL10008      ---------- ---------- ---------- ---------- ---------- ----------
AL20218      ---------- ---------- ---------- ---------- ---------- ----------
AL20219      ---------- ---------- ---------- ---------- ---------- ----------
AL20220      ---------- ---------- ---------- ---------- ---------- ----------

AL20223 ---------- ---------- ---------- ---------- ---------- ----------
AL20470 ---------- ---------- ---------- ---------- ---------- ----------
AL20471 ---------- ---------- ---------- ---------- ---------- ----------
AL20455 ---------- ---------- ---------- ---------- ---------- ----------

421
PHARMAQ001 IVGCLVPDHD PVYKVSIVPR GRALGVTMYL PEEDSYGYSR ERLESLISSM YGGRIAEALV
LF-89 IVGCLVPDHD PVYKVSIVPR GRALGVTMYL PEEDSYGYSR ERLESLISSM YGGRIAEALV
EM-90 IVGCLVPDHD PVYKVSIVPR GRALGVTMYL PEEDSYGYSR ERLESLISSM YGGRIAEALV
B1_32597 IVGCLVPDHD PVYKVSIVPR GRALGVTMYL PEEDSYGYSR ERLESLISSM YGGRIAEALV
A1_15972 IVGCLVPDHD PVYKVSIVPR GRALGVTMYL PEEDSYGYSR ERLESLISSM YGGRIAEALV
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20220 ---------- ---------- ---------- ---------- ---------- ----------
AL20223 ---------- ---------- ---------- ---------- ---------- ----------
AL20470 ---------- ---------- ---------- ---------- ---------- ----------
AL20471 ---------- ---------- ---------- ---------- ---------- ----------
AL20455 ---------- ---------- ---------- ---------- ---------- ----------

481
PHARMAQ001 FGVEKVTTGA SNDIEKASEV ARNMVTKWGL SERLGPILYG QEGGDPFGYG AGKGTPEFSD
LF-89 FGVEKVTTGA SNDIEKASEV ARNMVTKWGL SERLGPILYG QEGGDPFGYG AGKGTPEFSD
EM-90 FGVEKVTTGA SNDIEKASEV ARNMVTKWGL SERLGPILYG QEGGDPFGYG AGKGTPEFSD
B1_32597 FGVEKVTTGA SNDIEKASEV ARNMVTKWGL SERLGPILYG QEGGDPFGYG AGKGTPEFSD
A1_15972 FGVEKVTTGA SNDIEKASEV ARNMVTKWGL SERLGPILYG QEGGDPFGYG AGKGTPEFSD
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20220 ---------- ---------- ---------- ---------- ---------- ----------
AL20223 ---------- ---------- ---------- ---------- ---------- ----------
AL20470 ---------- ---------- ---------- ---------- ---------- ----------
AL20471 ---------- ---------- ---------- ---------- ---------- ----------
AL20455 ---------- ---------- ---------- ---------- ---------- ----------

541
PHARMAQ001 QTSVAIDEEV RQIIDRNYTR AESILINNRD ILDAMADALM VYETIDRDQV ADLMARRPVK
LF-89 QTSVAIDEEV RQIIDRNYTR AESILINNRD ILDAMADALM VYETIDRDQV ADLMARRPVK
EM-90 QTSVAIDEEV RQIIDRNYTR AESILINNRD ILDAMADALM VYETIDRDQV ADLMARRPVK
B1_32597 QTSVAIDEEV RQIIDRNYTR AESILIDNRD ILDAMADALM VYETIDREQV ADLMARRPVK
A1_15972 QTSVAIDEEV RQIIDRNYTR AESILINNRD ILDAMADALM VYETIDRDQV ADLMARRPVK
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20220 ---------- ---------- ---------- ---------- ---------- ----------
AL20223 ---------- ---------- ---------- ---------- ---------- ----------
AL20470 ---------- ---------- ---------- ---------- ---------- ----------
AL20471 ---------- ---------- ---------- ---------- ---------- ----------
AL20455 ---------- ---------- ---------- ---------- ---------- ----------

601
PHARMAQ001 APKDWDQPSD ESGSSASGDE LQPLDANINT DINETKSADQ ETDQGAPSPE IKGKPADDPT
LF-89 APKDWDQPSD ESGSSASGDE LQPLDANINT DINETKSADQ ETDQGAPSPE IKGKPADDPT
EM-90 APKDWDQPSD ESGSSASGDE LQPLDANINT DINETKSADQ ETDQGAPSPE IKGKPADDPT
B1_32597 APKDWDQPSD ESGSSASGDE LQPLDANINT DINDTKSADQ EIDQGAPSPE IKGKPADDPT
A1_15972 APKDWDQPSD ESGSSASGDE LQPLDANINT DINETKSADQ ETDQGAPSPE IKGKPADDPT
AL10016 ---------- ---------- ---------- ---------- ---------- ----------
AL10008 ---------- ---------- ---------- ---------- ---------- ----------
AL20218 ---------- ---------- ---------- ---------- ---------- ----------
AL20219 ---------- ---------- ---------- ---------- ---------- ----------
AL20220 ---------- ---------- ---------- ---------- ---------- ----------
AL20223 ---------- ---------- ---------- ---------- ---------- ----------

AL20470    ---------- ----------
AL20471    ---------- ----------
AL20455    ---------- ----------

661
PHARMAQ001  * (Seq. ID No. 53)
LF-89       * (Seq. ID No. 54)
EM-90       * (Seq. ID No. 55)
B1_32597    * (Seq. ID No. 56)
A1_15972    * (Seq. ID No. 57)
AL10016     - (Seq. ID No. 58)
AL10008     - (Seq. ID No. 59)
AL20218     - (Seq. ID No. 60)
AL20219     - (Seq. ID No. 61)
AL20220     - (Seq. ID No. 62)
AL20223     - (Seq. ID No. 63)
AL20470     - (Seq. ID No. 64)
AL20471     - (Seq. ID No. 65)
AL20455     - (Seq. ID No. 66)

The *FtsH* amino acid sequence alignment reveals that there are natural polymorphisms of the *FtsH* gene product between wild-type strains. However, all of the wild-type strains are virulent and therefore none of the differences between the wild-type sequences can be considered to affect virulence. PHARMAQ 001 has a methionine to isoleucine mutation at position 191 of the amino acid sequence which is not seen in any of the wild-type strains investigated, such as A1-15972, B1-32597, EM-90, and/or LF-89. The protein sequence of the *FtsH* gene product in PHARMAQ 001 is otherwise identical to that of wild-type strains including A1-15972, EM-90, and LF-89. The sequences of 13 wild-type strains were examined in the region of amino acid residues 152-274 of the *FtsH* gene product. All of the sequences were found to be identical in this region, but different to that of PHARMAQ 001.

It is clear from the alignments that *P. salmonis* gene products are very highly conserved in all of the strains investigated. As would be expected, there are natural polymorphisms in the genes wherein some of the wild-type strains have a sequence that is different from that of other wild-type strains. However, since all the strains except PHARMAQ 001 are virulent, these differences cannot contribute to the loss of virulence and consequent attenuated phenotype observed in PHARMAQ 001.

PHARMAQ 001 has mutations in the amino acid sequence of the *rpoD, FecR, ATP-grasp domain protein,* and *FtsH* gene products relative to the wild-type sequence, and also has an attenuated phenotype. These mutations are the only mutations observed in PHARMAQ 001 which lead to a significant alteration in the amino acid sequence of a protein, and they are not observed in any of the virulent strains investigated.

In a *P. salmonis* strain with an attenuated phenotype, if a mutation is observed in one of the *rpoD, FecR, ATP-grasp domain protein,* or *FtsH* genes which is also present in a virulent wild-type strain, then the mutation cannot be responsible for the attenuated phenotype. Mutations, and in particular attenuating mutations, in the *rpoD, FecR, ATP-grasp domain protein,* or *FtsH* genes are therefore only significant if they lead to a difference in the amino acid sequence of the gene product relative to the protein sequence of one of the virulent wild-type strains, such as A1-15972, B1-32597, EM-90, and/or LF-89.

### Example 3: Phylogeny of Piscirickettsia salmonis

To classify the *Piscirickettsia salmonis* isolates in Table 1, a simplified MLSA-scheme was used employing the genetic information within the arginine N-succinyltransferase (ast), glutamate-1-semialdehyde aminotransferase (hemL), L-serine dehydratase (sdhL), and UDP-glucose-4-epimerase (galE) genes for phylogenetic predictions (as annotated in the genome of A1-15972). Sequence information was obtained by standard PCR and sequencing. DNA sequences were assembled, quality checked and trimmed using Vector NTI^{®} software. Sequences for the strains A1-15972, B1-32597, LF-89 and EM-90 were retrieved from GenBank. Sequence information from the four genes for each strain were trimmed and concatenated before alignment.

Sequence alignments and phylogenetic predictions were performed in MEGA5, as described in Tamura et al. (Mol Biol Evol. 2011 Oct; 28(10): 2731-9), with the following parameters:
*Statistical Method:* Maximum Likelihood (100 Bootstrap Replications)
*Substitution Model:* Tamura-Nei model
*Rates*: Gamma distributed with Invariant sites (16 Discrete Gamma Categories)

Upon phylogenetic analysis of DNA sequences from the four genes above, two distinct and separate genotypes were identified. The two genotypes are here referred to as the LF-89 group and the EM-90 group. PHARMAQ 001 was found to group together with the EM-90 group. The LF-89 sequence used in the preparation of the phylogenetic tree was the genomic sequence deposited under GenBank accession no. AMGC00000000.1.

AL 20 223
B1-32597
AL 20 222
AL 20 220
AL20 471
LF-89
LF-89 group
AL 20 218
AL 10005
100
A1-15972
EM-90
EM-90 group
0.01

### Example 4: Avirulence of isolate PHARMAQ 001 when used as a live attenuated vaccine.

PHARMAQ 001 was cultivated in insect cell medium to an OD₆₀₀ₙₘ = 3.0 before the addition of a cryoprotectant and storage at -80 °C. The bacterial content was determined to be 2.6 × 10⁸ TCID₅₀/ml by end-point titration.

An ampoule of frozen isolate PHARMAQ 001 was thawed at room temperature and a 0.1ml dose containing 1.3 × 10⁷ TCID₅₀/ml was injected intraperitoneally into Atlantic salmon with an average weight of 25-30 grams.

The fish were held at 15°C for 44 days in freshwater. No fish died or showed any clinical signs of SRS.

Tissue samples (from head kidney and spleen) from vaccinated fish were taken every week for three weeks after vaccination. The presence of bacterial genomes in the tissue samples was analyzed by real time quantitative-PCR. The results are shown in Table 2.

**Table 2**

| **Days post-vaccination** | **Mean Ct values (9 fish per group)** | |
|---|---|---|
| | **head kidney** | **Spleen** |
| 7 | 28.54 | 28.72 |
| 14 | 30.17 | 29.42 |
| 21 | 31.69 | 29.80 |

As shown in Table 2, the presence of PHARMAQ 001 genomes was generally found to peak at 7 days post vaccination with Ct values around 28.

This experiment demonstrates that PHARMAQ 001 is avirulent and does not induce any symptoms of SRS. Therefore, PHARMAQ 001 is safe and suitable for use as an attenuated live vaccine.

### Example 5: Vaccination with PHARMAQ 001 and challenge with a virulent P. salmonis isolate

To examine the efficacy of the *P. salmonis* isolate PHARMAQ 001 as a vaccine against SRS, Rainbow trout (*Oncorhynchus mykiss*) with an average weight of 30 grams were injected with different doses of the vaccine isolate. The vaccine isolate was either preserved as a frozen vaccine and then diluted in PBS for use at a concentration of 2×10⁵ TCID₅₀, or as a lyophilized vaccine and then diluted in PBS for use at a concentration of 5.2×10⁶ TCID₅₀ per fish.

After an immunization period in fresh water for 504 degree days, the trout were challenged with a highly virulent wild-type strain of *P. salmonis.* The virulent wild-type strain was injected intraperitoneally and the fish were observed for 34 days.

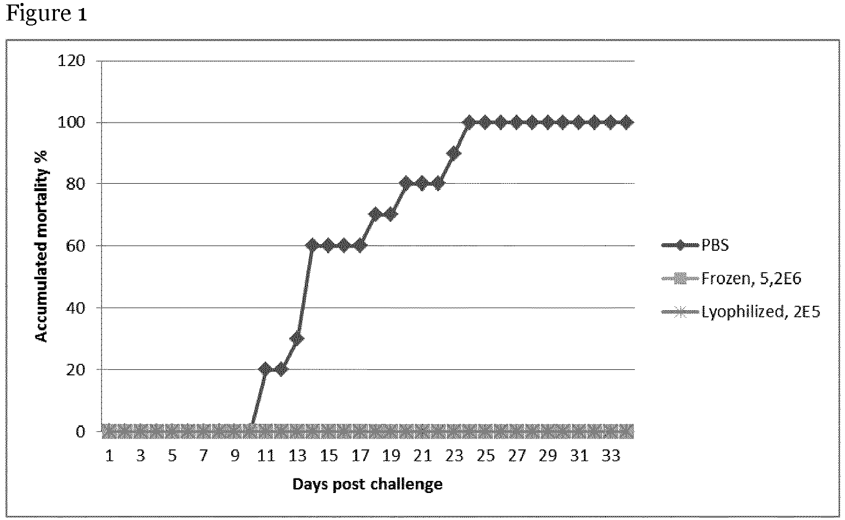

Figure 1 shows the accumulated mortality following challenge with a virulent *P*. *salmonis* strain.

The attenuated *P. salmonis* strain PHARMAQ 001 was found to work well as a live attenuated vaccine, and provided 100% protection against SRS.

In addition, the study shows that PHARMAQ 001 can be either frozen or lyophilized prior to being used as a vaccine.

### Example 6: Passage of attenuated P. salmonis strain PHARMAQ 001 in fish shows safety of the vaccine strain

To investigate the potential for reversion to virulence, the isolate PHARMAQ 001 was serially passaged through Atlantic salmon. The trial was performed in fresh water at 15°C. Atlantic salmon were injected with isolate PHARMAQ 001. Homogenates were prepared from head kidneys 7 days after injection, and new Atlantic salmon were injected with the homogenate.

After a further 7 days, homogenates were prepared from the head kidneys of the second fish, and further Atlantic salmon were injected with the homogenate.

No mortality or clinical signs of SRS were observed in any fish during the trial.

The liver from fish from each passage was homogenized, and homogenates were tested for the presence of live *P. salmonis* by plating onto CHAB agar plates. Bacteria were only detected after the first passage.

The bacterial loads in liver and spleen were investigated by measuring the presence of *P. salmonis* genomes by real time quantitative PCR one week after injection of PHARMAQ 001 or homogenate. The results (shown in Table 3) demonstrate that the bacterial loads in spleen and liver were reduced when head kidney homogenates were passaged from the first injected fish into passages 2 and 3 of fish.

**Table 3**

| | **Initial injection** | **Passage 2** | **Passage 3** |
|---|---|---|---|
| Liver | 28.9 | nd | nd |
| Spleen | 28.2 | 32.8 | nd |

| | | | |
|---|---|---|---|
| (nd=not detected) | | | |

This experiment shows that PHARMAQ 001 does not revert to a virulent strain after serial passage in Atlantic salmon. PHARMAQ 001 is therefore suitable for use as a live attenuated vaccine.

### Example 7: Culturing PHARMAQ 001 in spinner flask

Bacterial cultures were grown in ExCell Titer High medium from Sigma with no supplements. The cultures were incubated in ventilated spinner flasks at 75 rpm and 20°C, 2 passages after thawing. The growth was monitored by OD₆₀₀ₙₘ measurement.

**Table 4**

| **Step** | **Media** | **pH** | **Ventilation** | **Stirring** | **Temp** | **Vol. of Inoculum** | **Time** | **End OD₆₀₀** |
|---|---|---|---|---|---|---|---|---|
| 1. Spinner flask | ExCell TiterHigh | Not controlled | Ventilated cap | 75 rpm | 20 °C | 1% | 3 days | 3.3 |
| 2. Spinner flask | ExCell TiterHigh | Not controlled | Ventilated cap | 75 rpm | 20 °C | 1% | 2 days | 4.4 |

The results shown in Table 4 demonstrate the cultivation of *P. salmonis* in spinner flasks as the OD₆₀₀ reached 3-4 after 3-4 days of incubation.

### Example 8: Culturing PHARMAQ 001 in spinner flask

### P. salmonis strain PHARMAQ 001 was grown in spinner flasks in Sf-900 medium.

PHARMAQ 001 was harvested from the spinner flask and 20% skimmed milk in water (made from dry skimmed milk and heated for 15 minutes at 80°C) was added 1:1 to the *P. salmonis* culture to a final concentration of 10% skimmed milk. 2ml of this mixture (*P. salmonis* and 10% skimmed milk) was placed into 10ml glass vials and then freeze dried in a Labconco FreeZone Triad freeze dryer.

### Freeze drying cycle:

*Freezing*: 3 hours hold at -75°C
*Segment 1*: ramping rate: 0.1°C/min; holding time: 1 hour; shelf temperature: -40°C
*Segment 2*: ramping rate: 0.1°C/min; holding time: 8 hours; shelf temperature: -25°C
*Segment 3:* ramping rate: 0.1°C/min; holding time: 24 hours; shelf temperature: -10°C
*Segment 4*: ramping rate: 0.1°C/min; holding time: indefinite; shelf temperature: 4°C Vacuum was set to οµBar in step 1-4

The vials were sealed under vacuum and the process was stopped. The freeze dried materials were stored at 2-8 °C.

To determine the viability of the samples, titration (TCID₅₀/ml) was performed on the culture at harvest and after freeze drying. The freeze dried cake was rehydrated with 2ml PBS before titration.
Titer (TCID₅₀/ml) at harvest: 8.7 × 10⁹ cells/ml
Titer (TCID₅₀/ml) after freeze drying: 3.2 × 10⁷ cells/ml

Thus, after freeze drying, the samples are sufficiently viable for use as a vaccine.

### Example 8: Detecting PHARMAQ 001 strain

The attenuated live strain of *P. salmonis (rpoD*/ *FecR*/ *ATP-grasp domain protein*/ *FtsH)* has been shown to be well tolerated in healthy fish hosts and to colonise the host in a manner consistent with its utility as an effective vaccine to protect against SRS. It has also been demonstrated to elicit a specific immune response. The PHARMAQ 001 live attenuated *P. salmonis* strain has been found to be particularly effective for this purpose.

An assay was developed for identifying and detecting the PHARMAQ 001 strain of *P*. *salmonis.* The genome of the PHARMAQ 001 strain contains single nucleotide polymorphisms (SNP) in each of the *rpoD, FecR, ATP-grasp domain protein*, and *FtsH* genes, each of which results in a chemically significant alteration of the amino acid sequence of the resulting gene product. The assay involves typing each of these single nucleotide polymorphisms, and determining whether the wild-type or mutant allele is present.

Specifically, the assay involves using target-specific PCR primers to amplify the nucleic acid sequence in the region of the SNP. Separate probes specific for each of the wild-type and mutant alleles are included in the reaction mix. Each probe is labelled with a different detectable marker such as a fluorescent dye. In the example described below, the probe specific for the wild-type sequence was labelled with the FAM fluorophore, and the probe specific for the mutant sequence was labelled with the VIC fluorescent dye. An overview of the primers and probes used for each SNP analysis is shown in Table 5. In the present example, the SNP analysis was performed using the ABI PRISM^{®} 7900 HT Sequence Detection System.

**Table 5**

| **Target Gene** | **Primer/Probe** | **Sequence** | **Seq. ID No.** |
|---|---|---|---|
| rpoD | *rpoD SNP Detection Forward Primer* | GGACAATCCGGATTCCTGTACATAT | 67 |
| | *rpoD VProbe1-VIC Mutant Allele* | ACAAGCTTAAC**T**GCGTCTC | 68 |
| | *rpoD MProbe2-FAM Wild-Type Allele* | AAGCTTAAC**C**GCGTCTC | 69 |
| | *rpoD SNP Detection Reverse Primer* | GCCGAGTTCTTGGATCATTTGAC | 70 |
| ATP-grasp domain protein | *ATP-Grasp Domain Protein SNP Detection Forward Primer* | TGGCGATGGTGCGTCTAC | 71 |
| | *ATP-Grasp Domain Protein VProbei-VIC Mutant Allele* | CCATCAG**G**GAGCCGAG | 72 |
| | *ATP-Grasp Domain Protein MProbe2-FAM Wild-Type Allele* | CCATCAG**A**GAGCCGAG | 73 |
| | *ATP-Grasp Domain Protein SNP Detection Reverse Primer* | CGCCAATTGCACCTTGATGAAG | 74 |
| FtsH | *FtsH NP Detection Forward Primer* | CCAAAAGTTAGGCGGCAAAATTCC | 75 |
| | *FtsH VProbe1-VIC Mutant Allele* | TGGGCCAAC**T**ATCA | 76 |
| | *FtsH MProbe2-FAM Wild-Type Allele* | TGGGCCAAC**C**ATCA | 77 |
| | *FtsH SNP Detection Reverse Primer* | GCTAATAGCGTCTTACCTGTTCCA | 78 |

RNA was isolated from cultures of *P. salmonis,* including PHARMAQ 001 and wild type strains including the virulent starting strain. The samples were prepared as shown in Table 6. All tests were performed using QuantiTect Probe RT-PCR kit (Qiagen).

**Table 6**

| **Reagent** | **Final concentration** |
|---|---|
| 2X Master mix (Recommended by kit supplier, contains dNTPs, MgCl₂ (final concentration 4mM), HotStartTaq DNA Polymerase, and passive reference dye (ROX) | 1x |
| Forward primer | 900 nM (0.9 µl of 1.0 µM solution) |
| Reverse Primer | 900 nM (0.9 µl of 1.0 µM solution) |
| VProbe-VIC | 200 nM (0.2 µl of 1.0 µM solution) |
| MProbe-FAM | 175 nM (0.175 µl of 1.0 µM solution) |
| RT- Enzyme mix (QuantiTect) | 0.1 µl |
| Template | 1 pg to 1 µg per reaction |
| dH₂O | To 10 µl |

Samples were analysed in triplicates on 384-well plates. Each plate was subjected to a pre-read, for determination of background fluorescence in each well prior to the real-time RT-PCR step. The real-time RT-PCR was performed using standard enzymes and buffers, with the parameters shown in Table 7.

**Table 7**

| **Step** | | **Temperature** | **Time** | **Cycles** |
|---|---|---|---|---|
| | 1) Reverse transcription | 50°C | 30 minutes | 1 |
| | 2) DNA polymerase activation | 95°C | 15 minutes | 1 |
| | 3) Denaturation, Annealing and extension | 94°C | 15 seconds | 45 |
| | | 6o°C | 1 minute | |

All primers and probes were optimized to allow annealing and extension at 60°C. This temperature is also believed to be significant for the competition between the two probes in the SNP assay, as it leads to binding and cleavage of the correct probe as well as destabilization of the incorrect probe, depending on the SNP at the probe site.

After the real-time RT-PCR reaction had been performed, the plate was subjected to an end-point analysis, by performing a post-read of the fluorescence in each well, and comparing the result to the data stored from the pre-read. The results are shown in

Table 8 (in which a plus sign indicates a cycle threshold of less than or equal to 30 and a minus sign indicates no detectable fluorescent signal).

**Table 8**

| | rpoD | | ATP-grasp domain protein | | FtsH | |
|---|---|---|---|---|---|---|
| | *VProbe1-VIC Mutant Allele* | *MProbe2-FAM Wild-Type Allele* | *VProbe1-Vic Mutant Allele* | *MProbe2-FAM Wild-Type Allele* | *VProbe1-VIC Mutant Allele* | *MProbe2-FAM Wild-Type Allele* |
| PHARMAQ 001, attenuated vaccine strain | + | - | + | - | + | - |
| Virulent starting strain | - | + | - | + | - | + |
| Wild type *P.salmonis* strain A | - | + | - | + | - | + |
| Wild type *P.salmonis* strain B | - | + | - | + | - | + |
| Wild type *P.salmonis* strain C | - | + | - | + | - | + |

The assay clearly identified each of the mutant alleles in the PHARMAQ 001 strain, and also identified the presence of the wild-type allele in all wild type strains tested. For all tests, the discrimination between the two allelic variants was very good. The assay permits clear distinction between wild type and PHARMAQ 001 *P. salmonis* strains.

In order to address various issues and advance the art, the entirety of this disclosure shows by way of illustration various embodiments in which the claimed invention may be practiced and provide for an attenuated *P. salmonis* bacterium and an improved *P*. *salmonis* vaccine. They are presented only to assist in understanding and teach the claimed features. Various embodiments may suitably comprise, consist of, or consist essentially of, various combinations of the disclosed elements, components, features, parts, steps, means, etc.

## Claims

1. An attenuated *Piscirickettsia salmonis* bacterium comprising a mutation in the amino acid sequence of each of the *rpoD, FecR, ATP-grasp domain protein, and FtsH* gene products, the mutations comprising:
a) an arginine to cysteine mutation at position 473 of the *rpoD* gene product provided as Seq. ID No. 17;
b) a premature stop codon at the position corresponding to residue 83 of the *FecR* gene product provided as Seq. ID No. 26;
c) a serine to proline mutation at position 184 of *the ATP-grasp domain protein* gene product provided as Seq. ID No. 40; and,
d) a methionine to isoleucine mutation at position 191 of the *FtsH* gene product provided as Seq. ID No. 54.

2. An attenuated bacterium as claimed in claim 1, which is the strain PHARMAQ 001 deposited with the European Collection of Cell Cultures, Public health England, Culture Collections, Porton Down, Salisbury SP4 OJG, United Kingdom, on 09th October 2014 with accession number 14100901.

3. A live, attenuated vaccine composition comprising:
(a) an attenuated *Piscirickettsia salmonis* bacterium as claimed in any of claims 1-2; and
(b) a pharmaceutically acceptable carrier or diluent.

4. A live, attenuated vaccine composition as claimed in claim 3, in freeze-dried form.

5. A method of producing an attenuated bacterium as claimed in any of claims 1-2, the method comprising:
1) subjecting an initial population of *P. salmonis* bacteria to attenuating conditions to produce a putatively attenuated bacterial population;
2) identifying clones of the putatively attenuated bacterial population that have mutations in the amino acid sequences of all of the *rpoD, FecR, ATP-grasp domain protein,* and *FtsH* gene products; and then,
3) identifying and selecting clones that have mutations in the amino acid sequence of all of the *rpoD, FecR, ATP-grasp domain protein,* and *FtsH* gene products and that also exhibit reduced virulence relative to wild-type bacteria of the genus Piscirickettsia.

6. A live attenuated vaccine composition according to claim 3 or 4 for use in raising an immune response in a fish, the use comprising administering to the fish said vaccine composition comprising an attenuated *Piscirickettsia salmonis* bacterium as claimed in any of claims 1-2.

7. A live attenuated vaccine composition according to claim 3 or 4 for use in prevention of or treatment against Salmon Rickettsial Syndrome in a fish, the use comprising administering to a fish an immunologically-effective amount of said vaccine composition, said vaccine composition comprising an attenuated *Piscirickettsia salmonis* bacterium as claimed in any of claims 1-2.

8. An attenuated bacterium as claimed in any one of claims 1-2, for use in a method of vaccinating a fish.

9. An attenuated bacterium as claimed in any one of claims 1-2, for use in a method of vaccinating a fish against Salmon Rickettsial Syndrome.

## Patentansprüche

1. Abgeschwächtes *Piscirickettsia* salmonis-Bakterium, umfassend eine Mutation in der Aminosäuresequenz jedes der Genprodukte *rpoD, FecR, ATP-grasp-domain-Protein* und *FtsH,* wobei die Mutationen umfassen:
a) eine Arginin-zu-Cystein-Mutation an Position 473 des rpoD-Genprodukts, das als Seq. ID No. 17 bereitgestellt ist;
b) ein vorzeitiges Stoppcodon an der Position, die dem Rest 83 des FecR-Genprodukts entspricht, das als Seq. ID No. 26 bereitgestellt ist;
c) eine Serin-zu-Prolin-Mutation an Position 184 des *ATP-grasp-domain-Protein-Genprodukts,* das als Seq. ID No. 40 bereitgestellt ist; und
d) eine Methionin-zu-Isoleucin-Mutation an Position 191 des FtsH-Genprodukts, das als Seq. ID No. 54 bereitgestellt ist.

2. Abgeschwächtes Bakterium, wie in Anspruch 1 beansprucht, bei dem es sich um den Stamm PHARMAQ 001 handelt, der bei der European Collection of Cell Cultures, Public health England, Culture Collections, Porton Down, Salisbury SP4 OJG, Vereinigtes Königreich, am 09. Oktober 2014 mit der Zugangsnummer 14100901 hinterlegt wurde.

3. Abgeschwächte Lebendimpfstoffzusammensetzung, umfassend:
(a) ein abgeschwächtes *Piscirickettsia salmonis*-Bakterium, wie in einem der Ansprüche 1-2 beansprucht; und
(b) einen pharmazeutisch akzeptablen Träger oder ein pharmazeutisch akzeptables Verdünnungsmittel.

4. Abgeschwächte Lebendimpfstoffzusammensetzung, wie in Anspruch 3 beansprucht, in gefriergetrockneter Form.

5. Verfahren zur Herstellung eines abgeschwächten Bakteriums, wie in einem der Ansprüche 1-2 beansprucht, wobei das Verfahren umfasst:
1) Unterwerfen einer Ausgangspopulation von *P. salmonis-*Bakterien abschwächenden Bedingungen, um eine vermeintlich abgeschwächte Bakterienpopulation zu erzeugen;
2) Identifizierung von Klonen der vermeintlich abgeschwächten Bakterienpopulation, die Mutationen in den Aminosäuresequenzen aller *rpoD-, FecR-, ATP-grasp-domain-Protein-* und *FtsH*-Genprodukte aufweisen; und dann
3) Identifizierung und Auswahl von Klonen, die Mutationen in der Aminosäuresequenz aller *rpoD-, FecR-, ATP-grasp-domain-Protein-* und *FtsH*-Genprodukte aufweisen und die auch eine verringerte Virulenz im Vergleich zu Wildtyp-Bakterien der Gattung Piscirickettsia zeigen.

6. Abgeschwächte Lebendimpfstoffzusammensetzung nach Anspruch 3 oder 4 zur Verwendung bei der Erzeugung einer Immunantwort in einem Fisch, wobei die Verwendung die Verabreichung der Impfstoffzusammensetzung an den Fisch umfasst, die ein abgeschwächtes *Piscirickettsia salmonis-*Bakterium, wie in einem der Ansprüche 1-2 beansprucht, umfasst.

7. Abgeschwächte Lebendimpfstoffzusammensetzung nach Anspruch 3 oder 4 zur Verwendung bei der Vorbeugung oder Behandlung des Lachs-Rickettsien-Syndroms bei einem Fisch, wobei die Verwendung die Verabreichung einer immunologisch wirksamen Menge der Impfstoffzusammensetzung an einen Fisch umfasst, wobei die Impfstoffzusammensetzung ein abgeschwächtes *Piscirickettsia salmonis*-Bakterium, wie in einem der Ansprüche 1-2 beansprucht, umfasst.

8. Abgeschwächtes Bakterium, wie in einem der Ansprüche 1-2 beansprucht, zur Verwendung in einem Verfahren zur Impfung eines Fisches.

9. Abgeschwächtes Bakterium, wie in einem der Ansprüche 1-2 beansprucht, zur Verwendung in einem Verfahren zur Impfung eines Fisches gegen das Salmon-Rickettsial-Syndrom.

## Revendications

1. Bactérie *Piscirickettsia salmonis* atténuée comprenant une mutation dans la séquence d'acides aminés dans chacun des produits géniques de *rpoD, FecR, protéine à domaine ATP-grasp,* et *FtsH,* les mutations comprenant :
a) une mutation d'arginine en cystéine à la position 473 du produit génique de *rpoD* se présentant comme la SEQ ID NO : 17 ;
b) un codon non-sens prématuré à la position correspondant au résidu 83 du produit génique de *FecR* se présentant comme la SEQ ID NO : 26 ;
c) une mutation de sérine en proline à la position 184 du produit génique de *protéine à domaine ATP-grasp* se présentant comme la SEQ ID NO : 40 ; et
d) une mutation de méthionine en isoleucine à la position 191 du produit génique de *FtsH* se présentant comme la SEQ ID NO : 54.

2. Bactérie atténuée selon la revendication 1, qui est la souche PHARMAQ 001 déposée à l'European Collection of Cell Cultures, Public Health England, Culture Collections, Porton Down, Salisbury SP4 OJG, Royaume-Uni, le 9 octobre 2014 avec le numéro d'accès 14100901.

3. Composition de vaccin atténué vivant comprenant :
(a) une bactérie *Piscirickettsia salmonis* atténuée selon l'une quelconque des revendications 1 et 2 ; et
(b) un véhicule ou diluant pharmaceutiquement acceptable.

4. Composition de vaccin atténué vivant selon la revendication 3, sous forme lyophilisée.

5. Procédé de production d'une bactérie atténuée selon l'une quelconque des revendications 1 et 2, le procédé comprenant :
1) la soumission d'une population initiale de bactéries *P. salmonis* à des conditions d'atténuation pour produire une population bactérienne putativement atténuée ;
2) l'identification de clones de la population bactérienne putativement atténuée qui ont des mutations dans les séquences d'acides aminés de tous les produits géniques de *rpoD, FecR, protéine à domaine ATP-grasp,* et *FtsH ;* et ensuite
3) l'identification et la sélection de clones qui ont des mutations dans la séquence d'acides aminés de tous les produits géniques de *rpoD, FecR, protéine à domaine ATP-grasp,* et *FtsH* et qui présentent aussi une virulence réduite par rapport aux bactéries de type sauvage du genre Piscirickettsia.

6. Composition de vaccin atténué vivant selon la revendication 3 ou 4, pour une utilisation dans l'augmentation d'une réponse immunitaire chez un poisson, l'utilisation comprenant l'administration au poisson de ladite composition de vaccin comprenant une bactérie *Piscirickettsia salmonis* atténuée selon l'une quelconque des revendications 1 et 2.

7. Composition de vaccin atténué vivant selon la revendication 3 ou 4, pour une utilisation dans la prévention ou le traitement du syndrome rickettsien du saumon chez un poisson, l'utilisation comprenant l'administration à un poisson d'une quantité immunologiquement efficace de ladite composition de vaccin, ladite composition de vaccin comprenant une bactérie *Piscirickettsia salmonis* atténuée selon l'une quelconque des revendications 1 et 2.

8. Bactérie atténuée selon l'une quelconque des revendications 1 et 2, pour une utilisation dans une méthode de vaccination d'un poisson.

9. Bactérie atténuée selon l'une quelconque des revendications 1 et 2, pour une utilisation dans une méthode de vaccination d'un poisson contre le syndrome rickettsien du saumon.
